# EUROPEAN PATENT SPECIFICATION

(11) **EP 2 455 475 B1**
(45) Date of publication and mention of the grant of the patent: **03.01.2018**
(21) Application number: 12155889.4
(22) Date of filing: 30.01.2008
(51) Int. Cl.: C12N 15/82, C12N 9/02, A01H 5/08

(54) **Disease resistant plants**
Krankheitsresistente Pflanzen
Plantes résistantes aux maladies

(30) Priority: 01.02.2007 WO PCT/EP2007/050976
(43) Date of publication of application: 23.05.2012
(62) Divisional of application: 08707413.4
(73) Proprietor: Enza Zaden Beheer B.V., 1602 DB Enkhuizen (NL)
(72) Inventor: Van Damme, Mireille Maria Augusta, 6706 LG Wageningen (NL); Van Den Ackerveken, Augustinus Franciscus J.M., 3438 PC Nieuwegein (NL)
(74) Representative: van Kooij, Adriaan

(56) References cited:
- WO-A-00/78981
- WO-A-01/61021
- WO-A-2006/032707
- DAMME VAN M ET AL: "IDENTIFICATION OF ARABIDOPSIS LOCI REQUIRED FOR SUSCEPTIBILITY TO THE DOWNY MILDEW PATHOGEN HYALOPERONOSPORA PARASITICA", MOLECULAR PLANT-MICROBE INTERACTIONS, APS PRESS, ST. PAUL, MN, US, vol. 18, no. 6, June 2005 (2005-06), pages 583-592, XP008053420, ISSN: 0894-0282
- DATABASE EMBL [Online] 15 April 2002 (2002-04-15), "Arabidopsis thaliana flavanone 3-hydroxylase-like protein (At5g24530) mRNA, complete cds.", XP002454001, retrieved from EBI accession no. EMBL:AY081455 Database accession no. AY081455
- SKADHAUGE B ET AL: "The role of the barley testa layer and its flavonoid content in resistance to Fusarium infections", HEREDITAS, LUND, SE, vol. 126, no. 2, 1997, pages 147-160, XP002081490, ISSN: 0018-0661
- CHO ET AL: "Constitutive expression of the Flavanone 3-hydroxylase gene related to pathotype-specific ascochyta blight resistance in Cicer arietinum L", PHYSIOLOGICAL AND MOLECULAR PLANT PATHOLOGY, ACADEMIC PRESS LTD, GB, vol. 67, no. 2, August 2005 (2005-08), pages 100-107, XP005265989, ISSN: 0885-5765
- ARDI R ET AL: "Involvement of epicatechin biosynthesis in the activation of the mechanism of resistance of avocado fruits to Colletotrichum gloeosporioides", PHYSIOLOGICAL AND MOLECULAR PLANT PATHOLOGY, vol. 53, no. 5-6, November 1998 (1998-11), pages 269-285, XP002453991, ISSN: 0885-5765
- MOSHER REBECCA A ET AL: "A comprehensive structure-function analysis of Arabidopsis SNI1 defines essential regions and transcriptional repressor activity.", THE PLANT CELL JUL 2006, vol. 18, no. 7, July 2006 (2006-07), pages 1750-1765, XP002453995, ISSN: 1040-4651
- BALASS M ET AL: "IDENTIFICATION OF A CONSTITUTIVE 45 KDA SOLUBLE PROTEIN ASSOCIATED WITH RESISTANCE TO DOWNY MILDEW IN MUSKMELON (CUCUMIS MELO L.) LINEPI 124111F", PHYSIOLOGICAL AND MOLECULAR PLANT PATHOLOGY, ACADEMIC PRESS LTD, GB, vol. 41, 1 January 1992 (1992-01-01), pages 387-396, XP000926482, ISSN: 0885-5765, DOI: 10.1016/0885-5765(92)90051-V
- L. PERCHEPIED ET AL: "Relationship Between Loci Conferring Downy Mildew and Powdery Mildew Resistance in Melon Assessed by Quantitative Trait Loci Mapping", PHYTOPATHOLOGY, vol. 95, no. 5, 1 May 2005 (2005-05-01), pages 556-565, XP55022414, ISSN: 0031-949X, DOI: 10.1094/PHYTO-95-0556

### SEQUENCE LISTING

<110> Enza Zaden Beheer B.V.
   VAN DAMME, Mireille Maria Augusta
   VAN DEN ACKERVEKEN, Augustinus Franciscus Johannes Maria
<120> DISEASE RESISTANT PLANTS
<130> 4/2MG91/24C
<150> PCT/EP2007/050976
   <151> 2007-02-01
<160> 98
<170> PatentIn version 3.3
<210> 1
   <211> 33
   <212> DNA
   <213> Artificial
<220>
   <223> forward primer
<400> 1
   ttctgggatc caatggcggc aaagcttgat atc 33
<210> 2
   <211> 39
   <212> DNA
   <213> Artificial
<220>
   <223> reverse primer
<400> 2
   gatatatgaa ttcttagttg tttagaaaat tctcgaggc 39
<210> 3
   <211> 33
   <212> DNA
   <213> Artificial
<220>
   <223> RNAiDMR6F
<400> 3
   aaaaagcagg ctgaccgtcc acgtctctct gaa 33
<210> 4
   <211> 33
   <212> DNA
   <213> Artificial
<220>
   <223> RANiDMR6R
<400> 4
   agaaagctgg gtgaaacgat gcgaccgata gtc 33
<210> 5
   <211> 29
   <212> DNA
   <213> Artificial
<220>
   <223> attB1
<400> 5
   ggggacaagt ttgtacaaaa aagcaggct 29
<210> 6
   <211> 29
   <212> DNA
   <213> Artificial
<220>
   <223> attB2
<400> 6
   ggggaccact ttgtacaaga aagctgggt 29
<210> 7
   <211> 26
   <212> DNA
   <213> Artificial
<220>
   <223> internal primer for At5G24530
<400> 7
   gagaagtggg atttaaaata gaggaa 26
<210> 8
   <211> 22
   <212> DNA
   <213> Artificial
<220>
   <223> QDMR6F
<400> 8
   tgtcatcaac ataggtgacc ag 22
<210> 9
   <211> 22
   <212> DNA
   <213> Artificial
<220>
   <223> QDMR6R
<400> 9
   cgatagtcac ggattttctg tg 22
<210> 10
   <211> 20
   <212> DNA
   <213> Artificial
<220>
   <223> QAt1g114880F
<400> 10
   ctcaaggaga atggtccaca 20
<210> 11
   <211> 20
   <212> DNA
   <213> Artificial
<220>
   <223> QAt1g14880R
<400> 11
   cgacttggcc aaatgtgata 20
<210> 12
   <211> 21
   <212> DNA
   <213> Artificial
<220>
   <223> QAt4g14365F
<400> 12
   tggttttctg aggcatgtaa a 21
<210> 13
   <211> 20
   <212> DNA
   <213> Artificial
<220>
   <223> QAtfg14365R
<400> 13
   agtgcaggaa cattggttgt 20
<210> 14
   <211> 20
   <212> DNA
   <213> Artificial
<220>
   <223> QACD6F
<400> 14
   tggacagttc tggagcagat 20
<210> 15
   <211> 18
   <212> DNA
   <213> Artificial
<220>
   <223> QACD6R
<400> 15
   caactcctcc gctgtgag 18
<210> 16
   <211> 23
   <212> DNA
   <213> Artificial
<220>
   <223> QPR-5F
<400> 16
   ggcaaatatc tccagtattc aca 23
<210> 17
   <211> 22
   <212> DNA
   <213> Artificial
<220>
   <223> QPR-5R
<400> 17
   ggtagggcaa ttgttcctta ga 22
<210> 18
   <211> 20
   <212> DNA
   <213> Artificial
<220>
   <223> QPR-2F
<400> 18
   aaggagctta gcctcaccac 20
<210> 19
   <211> 20
   <212> DNA
   <213> Artificial
<220>
   <223> QPR-2R
<400> 19
   gagggaagca agaatggaac 20
<210> 20
   <211> 20
   <212> DNA
   <213> Artificial
<220>
   <223> QPR-1F
<400> 20
   gaacacgtgc aatggagttt 20
<210> 21
   <211> 21
   <212> DNA
   <213> Artificial
<220>
   <223> QPR-1R
<400> 21
   ggttccacca ttgttacacc t 21
<210> 22
   <211> 19
   <212> DNA
   <213> Artificial
<220>
   <223> QACT2F
<400> 22
   aatcacagca cttgcacca 19
<210> 23
   <211> 20
   <212> DNA
   <213> Artificial
<220>
   <223> QACT2R
<400> 23
   gagggaagca agaatggaac 20
<210> 24
   <211> 19
   <212> DNA
   <213> Artificial
<220>
   <223> Lsat-dmr6-fw1
<400> 24
   gatcaaggtc aacacatgg 19
<210> 25
   <211> 20
   <212> DNA
   <213> Artificial
<220>
   <223> Lsat_dmr6_fw2
<400> 25
   tcaaccatta cccagtgtgc 20
<210> 26
   <211> 20
   <212> DNA
   <213> Artificial
<220>
   <223> dmr6-deg₋fw1B
<400> 26
   ttccaggtda ttaaycaygg 20
<210> 27
   <211> 20
   <212> DNA
   <213> Artificial
<220>
   <223> dmr6_deg_fw2B
<400> 27
   cataaytgga grgaytayct 20
<210> 28
   <211> 20
   <212> DNA
   <213> Artificial
<220>
   <223> dmr6_deg_fw3b
<400> 28
   garcaaggrc arcayatggc 20
<210> 29
   <211> 20
   <212> DNA
   <213> Artificial
<220>
   <223> dmr6_deg_fw4
<400> 29
   aatcctcctt chttcaagga 20
<210> 30
   <211> 20
   <212> DNA
   <213> Artificial
<220>
   <223> dmr6_deg_rv3B
<400> 30
   agtgcattkg ggtchgtrtg 20
<210> 31
   <211> 20
   <212> DNA
   <213> Artificial
<220>
   <223> dmr6_deg_rv4
<400> 31
   aatgttratg acaaargcat 20
<210> 32
   <211> 20
   <212> DNA
   <213> Artificial
<220>
   <223> dmr6_deg_rv5
<400> 32
   gccatrtgyt gyccttgytc 20
<210> 33
   <211> 20
   <212> DNA
   <213> Artificial
<220>
   <223> Cuc-dmr6-rv1
<400> 33
   tccggacatt gaaacttgtg 20
<210> 34
   <211> 20
   <212> DNA
   <213> Artificial
<220>
   <223> Cuc_dmr6_rv2
<400> 34
   tcaaagaact gcttgccaac 20
<210> 35
   <211> 20
   <212> DNA
   <213> Artificial
<220>
   <223> Cuc-dmr6-fw1
<400> 35
   cgcactcacc attctccttc 20
<210> 36
   <211> 19
   <212> DNA
   <213> Artificial
<220>
   <223> Cuc_dmr6_fw2
<400> 36
   ggcctccaag tcctcaaag 19
<210> 37
   <211> 25
   <212> DNA
   <213> Artificial
<220>
   <223> IND_MOP9 Fw
<400> 37
   tttgggaaca gaaaaagttg gaggt 25
<210> 38
   <211> 25
   <212> DNA
   <213> Artificial
<220>
   <223> IND_MOP9 Rv
<400> 38
   catattcaaa agggaaaatc ccaga 25
<210> 39
   <211> 25
   <212> DNA
   <213> Artificial
<220>
   <223> IND_K16H17 Fw
<400> 39
   tggggttgtg gtttattctg ttgac 25
<210> 40
   <211> 26
   <212> DNA
   <213> Artificial
<220>
   <223> IND_K16H17 Rv
<400> 40
   tggccaatag tagttgatac gcaaga 26
<210> 41
   <211> 25
   <212> DNA
   <213> Artificial
<220>
   <223> IND_T4C12 Fw
<400> 41
   tctcgggtaa gacacaagtc gagat 25
<210> 42
   <211> 25
   <212> DNA
   <213> Artificial
<220>
   <223> IND_T4C12 Rv
<400> 42
   tattccaact tgcgacgtag agcat 25
<210> 43
   <211> 24
   <212> DNA
   <213> Artificial
<220>
   <223> IND_T11H3 Fw
<400> 43
   ccaattgggt tatttacttc gatt 24
<210> 44
   <211> 24
   <212> DNA
   <213> Artificial
<220>
   <223> IND_T11H3 Rv
<400> 44
   cggcttttaa caacatattt tcca 24
<210> 45
   <211> 18
   <212> DNA
   <213> Artificial
<220>
   <223> IND_F21J6 fw primer
<400> 45
   aacacatcac caagatga 18
<210> 46
   <211> 25
   <212> DNA
   <213> Artificial
<220>
   <223> IND_F21J6 rv primer
<400> 46
   cctctgcccc aagaaatatt gagat 25
<210> 47
   <211> 24
   <212> DNA
   <213> Artificial
<220>
   <223> M450 fw
<400> 47
   agctttgtat ggtagtgcca atga 24
<210> 48
   <211> 24
   <212> DNA
   <213> Artificial
<220>
   <223> M450 Rv
<400> 48
   gcggtatacg ggggttaaaa tcta 24
<210> 49
   <211> 22
   <212> DNA
   <213> Artificial
<220>
   <223> M490 Fw
<400> 49
   atggccaacc actctttgtt ac 22
<210> 50
   <211> 22
   <212> DNA
   <213> Artificial
<220>
   <223> M490 Rv
<400> 50
   acaagcaaga agaacagcga ag 22
<210> 51
   <211> 24
   <212> DNA
   <213> Artificial
<220>
   <223> M525 Fw
<400> 51
   gaaatttggt tgttggcatt tatc 24
<210> 52
   <211> 25
   <212> DNA
   <213> Artificial
<220>
   <223> M525 Rv
<400> 52
   tcaagatctt catattctca ttcca 25
<210> 53
   <211> 25
   <212> DNA
   <213> Artificial
<220>
   <223> M545 Fw
<400> 53
   cagctgaagt atgtttcatc ccttt 25
<210> 54
   <211> 24
   <212> DNA
   <213> Artificial
<220>
   <223> M545 Rv
<400> 54
   cttgcaattg ttgggactag gtaa 24
<210> 55
   <211> 24
   <212> DNA
   <213> Artificial
<220>
   <223> M555 Fw
<400> 55
   tcactaacca gtgaaaaagg ttgc 24
<210> 56
   <211> 24
   <212> DNA
   <213> Artificial
<220>
   <223> M555 Rv
<400> 56
   tatacagcga atagcaaagc caag 24
<210> 57
   <211> 24
   <212> DNA
   <213> Artificial
<220>
   <223> M470 Fw
<400> 57
   ccgcgagtgt aatatatctc tcct 24
<210> 58
   <211> 24
   <212> DNA
   <213> Artificial
<220>
   <223> M470 Rv
<400> 58
   cagtttaacg catgaagtgc tagt 24
<210> 59
   <211> 24
   <212> DNA
   <213> Artificial
<220>
   <223> M590 Fw
<400> 59
   gcatcatttg taccgtactg agtc 24
<210> 60
   <211> 24
   <212> DNA
   <213> Artificial
<220>
   <223> M590 Rv
<400> 60
   tagtggatac tctgtccctg aggt 24
<210> 61
   <211> 1026
   <212> DNA
   <213> Arabidopsis thaliana
<400> 61
   atggcggcaa agctgatatc caccggtttc cgtcatacta ctttgccgga aaactatgtc      60
   cggccaatct ccgaccgtcc acgtctctct gaagtctctc aactcgaaga tttccctctc     120
   atcgatctct cttccactga tcgatctttt ctcatccaac aaatccacca agcttgtgcc     180
   cgattcggat tttttcaggt cataaatcac ggagttaaca aacaaataat agatgagatg     240
   gtgagtgttg cgcgtgagtt ctttagcatg tctatggaag aaaaaatgaa gctatattca     300
   gacgatccaa cgaagacaac aagattatcg acgagcttca atgtgaagaa agaagaagtc     360
   aacaattgga gagactatct aagactccat tgttatccta tccacaagta tgtcaatgag     420
   tggccgtcaa accctccttc tttcaaggaa atagtaagta aatacagtag agaagtaaga     480
   gaagtgggat ttaaaataga ggaattaata tcagagagct taggtttaga aaaagattac     540
   atgaagaaag tgcttggtga acaaggtcaa cacatggcag tcaactatta tcctccatgt     600
   cctgaacctg agctcactta cggtttacct gctcataccg acccaaacgc cctaaccatt     660
   cttcttcaag acactactgt ttgcggtctc cagatcttga tcgacggtca gtggttcgcc     720
   gttaatccac atcctgatgc ttttgtcatc aacataggtg accagttaca ggcattaagt     780
   aatggagtat acaaaagtgt ttggcatcgc gctgtaacaa acacagaaaa tccgagacta     840
   tcggtcgcat cgtttctgtg cccagctgac tgtgctgtca tgagcccggc caagcccttg     900
   tgggaagctg aggacgatga aacgaaacca gtctacaaag atttcactta tgcagagtat     960
   tacaagaagt tttggagtag gaatctggac caagaacatt gcctcgagaa ttttctaaac    1020
   aactaa                                                               1026
<210> 62
   <211> 341
   <212> PRT
   <213> Arabidopsis thaliana
<400> 62
   Met Ala Ala Lys Leu Ile Ser Thr Gly Phe Arg His Thr Thr Leu Pro
   1               5                   10                  15
   Glu Asn Tyr Val Arg Pro Ile Ser Asp Arg Pro Arg Leu Ser Glu Val
               20                  25                  30
   Ser Gln Leu Glu Asp Phe Pro Leu Ile Asp Leu Ser Ser Thr Asp Arg
           35                  40                  45
   Ser Phe Leu Ile Gln Gln Ile His Gln Ala Cys Ala Arg Phe Gly Phe
       50                  55                  60
   Phe Gln Val Ile Asn His Gly Val Asn Lys Gln Ile Ile Asp Glu Met
   65                  70                  75                  80
   Val Ser Val Ala Arg Glu Phe Phe Ser Met Ser Met Glu Glu Lys Met
                   85                  90                  95
   Lys Leu Tyr Ser Asp Asp Pro Thr Lys Thr Thr Arg Leu Ser Thr Ser
               100                 105                 110
   Phe Asn Val Lys Lys Glu Glu Val Asn Asn Trp Arg Asp Tyr Leu Arg
           115                 120                 125
   Leu His Cys Tyr Pro Ile His Lys Tyr Val Asn Glu Trp Pro Ser Asn
       130                 135                 140
   Pro Pro Ser Phe Lys Glu Ile Val Ser Lys Tyr Ser Arg Glu Val Arg
   145                 150                 155                 160
   Glu Val Gly Phe Lys Ile Glu Glu Leu Ile Ser Glu Ser Leu Gly Leu
                   165                 170                 175
   Glu Lys Asp Tyr Met Lys Lys Val Leu Gly Glu Gln Gly Gln His Met
               180                 185                 190
   Ala Val Asn Tyr Tyr Pro Pro Cys Pro Glu Pro Glu Leu Thr Tyr Gly
           195                 200                 205
   Leu Pro Ala His Thr Asp Pro Asn Ala Leu Thr Ile Leu Leu Gln Asp
       210                 215                 220
   Thr Thr Val Cys Gly Leu Gln Ile Leu Ile Asp Gly Gln Trp Phe Ala
   225                 230                 235                 240
   Val Asn Pro His Pro Asp Ala Phe Val Ile Asn Ile Gly Asp Gln Leu
                   245                 250                 255
   Gln Ala Leu Ser Asn Gly Val Tyr Lys Ser Val Trp His Arg Ala Val
               260                 265                 270
   Thr Asn Thr Glu Asn Pro Arg Leu Ser Val Ala Ser Phe Leu Cys Pro
           275                 280                 285
   Ala Asp Cys Ala Val Met Ser Pro Ala Lys Pro Leu Trp Glu Ala Glu
       290                 295                 300
   Asp Asp Glu Thr Lys Pro Val Tyr Lys Asp Phe Thr Tyr Ala Glu Tyr
   305                 310                 315                 320
   Tyr Lys Lys Phe Trp Ser Arg Asn Leu Asp Gln Glu His Cys Leu Glu
                   325                 330                 335
   Asn Phe Leu Asn Asn
               340
<210> 63
   <211> 242
   <212> PRT
   <213> Aquilegia sp.
<400> 63
   Met Glu Ser Ser Asn Val Leu Leu Thr Gly Thr Arg His Ser Asn Leu
   1               5                   10                  15
   Pro Glu Asn Tyr Val Arg Ser Val Ser Asp Arg Pro Arg Leu Ser Glu
               20                  25                  30
   Val Lys Asp Cys Glu Asn Val Pro Val Ile Asp Leu Ser Val Ala Asp
           35                  40                  45
   Glu Ser Leu Leu Ala Gln Gln Ile Gly Asn Ala Cys Lys Ser His Gly
       50                  55                  60
   Phe Phe Gln Val Ile Asn His Gly Val Asn Ser Glu Leu Val Glu Lys
   65                  70                  75                  80
   Met Met Glu Ile Ser His Glu Phe Phe His Leu Pro Leu Asp Val Lys
                   85                  90                  95
   Met Gln Phe Tyr Ser Asp Asp Pro Thr Lys Thr Met Arg Leu Ser Thr
               100                 105                 110
   Ser Phe Asn Leu Lys Lys Glu Ser Val His Asn Trp Arg Asp Tyr Leu
   115 120 125
   Arg Leu His Cys His Pro Ile Glu Lys Tyr Val Gln Glu Trp Pro Ser
   130 135 140
   Val Pro Ser Thr Phe Lys Asp Val Val Ala Thr Tyr Cys Lys Glu Val
   145 150 155 160
   Arg Lys Leu Gly Leu Arg Leu Leu Gly Ser Ile Ser Leu Ser Leu Gly
   165 170 175
   Leu Glu Glu Asp Tyr Ile Glu Lys Val Leu Gly Asp Gln Gly Gln His
   180 185 190
   Met Ala Val Asn Tyr Tyr Pro Pro Cys Pro Glu Pro Glu Leu Thr Tyr
   195 200 205
   Gly Leu Pro Arg His Thr Asp Pro Asn Thr Ile Thr Ile Leu Leu Gln
   210 215 220
   Gly Gln Glu Val Ala Gly Leu Gln Val Leu His Asn Gly Lys Trp Val
   225 230 235 240
   Ala Val
<210> 64
   <211> 337
   <212> PRT
   <213> Citrus sinensis
<400> 64
   Met Asp Thr Lys Val Leu Ser Ser Gly Ile Arg Tyr Thr Asn Leu Pro
   1               5                   10                  15
   Glu Gly Tyr Val Arg Pro Glu Ser Glu Arg Pro Asn Leu Ser Glu Val
               20                  25                  30
   Ser Glu Cys Lys Asn Val Pro Val Ile Asp Leu Ala Cys Asp Asp Arg
           35                  40                  45
   Ser Leu Ile Val Gln Gln Val Ala Asp Ala Cys Lys Asn Tyr Gly Phe
       50                  55                  60
   Phe Gln Ala Ile Asn His Glu Val Pro Leu Glu Thr Val Glu Arg Val
   65                  70                  75                      80
   Leu Glu Val Ala Lys Glu Phe Phe Asn Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Asn Lys Glu Lys Val His Asn Trp Arg Asp Tyr Leu Arg
           115                 120                 125
   Leu His Cys Tyr Pro Leu Asp Lys Tyr Val Pro Glu Trp Pro Ser Asn
       130                 135                 140
   Pro Ser Thr Phe Lys Glu Phe Val Ser Thr Tyr Cys Ser Glu Val Arg
   145                 150                 155                 160
   Gly Leu Gly Tyr Arg Val Leu Glu Leu Ile Ser Glu Ser Leu Gly Leu
                   165                 170                 175
   Glu Lys Asp Tyr Ile Lys Lys Val Leu Gly Glu Gln Gly Gln His Met
               180                 185                 190
   Ala Val Asn Phe Tyr Pro Pro Cys Pro Glu Pro Glu Leu Thr Tyr Gly
           195                 200                 205
   Leu Pro Gly His Thr Asp Pro Asn Ala Leu Thr Ile Leu Leu Gln Asp
       210                 215                 220
   Leu Glu Val Ala Gly Leu Gln Val Leu Lys Asp Asp Lys Trp Val Ala
   225                 230                 235                 240
   Val Asn Pro Leu Pro Asn Ala Phe Val Ile Asn Ile Gly Asp Gln Leu
                   245                 250                 255
   Gln Ala Leu Ser Asn Gly Arg Tyr Lys Ser Val Trp His Arg Ala Ile
               260                 265                 270
   Val Asn Ala Glu Lys Ala Arg Met Ser Val Ala Ser Phe Leu Cys Pro
           275                 280                 285
   Asn Asn Asp Ala Met Ile Ser Pro Pro Lys Ala Leu Thr Glu Asp Gly
       290                 295                 300
   Ser Gly Ala Val Tyr Arg Asp Phe Thr Tyr Ala Glu Tyr Tyr Ser Lys
   305                 310                 315                 320
   Phe Trp Ser Arg Asn Leu Asp Gln Glu His Cys Leu Glu Leu Phe Lys
                   325                 330                 335
   Asn
<210> 65
   <211> 337
   <212> PRT
   <213> Coffea canephora
<400> 65
   Met Glu Thr Lys Val Ile Ser Ser Gly Ile Lys Tyr Thr Ser Leu Pro
   1               5                   10                  15
   Glu Ser Tyr Val Arg Pro Glu Ser Glu Arg Pro Arg Leu Ser Glu Val
               20                  25                  30
   Ser Asp Cys Gln Asn Val Pro Val Val Asp Leu Gly Phe Gly Asp Arg
           35                  40                  45
   Asn Leu Met Val Arg Gln Ile Gly Asp Ala Cys Arg Asp Tyr Gly Phe
       50                  55                  60
   Phe Gln Val Ile Asn His Gly Val Ser Lys Asp Ala Val Asp Lys Met
   65                  70                  75                  80
   Leu Glu Thr Ala Thr Glu Phe Phe Ser Leu Pro Val Glu Glu Lys Leu
                   85                  90                  95
   Lys Leu Tyr Ser Asp Asp Pro Ser Lys Thr Thr Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Val Pro Glu Trp Pro Ser Asn
       130                 135                 140
   Pro Pro Ser Phe Lys Glu Met Val Ser Asn Tyr Cys Val Gln Ile Arg
   145                 150                 155                 160
   Glu Leu Gly Leu Arg Leu Glu Glu Ala Ile Ala Glu Ser Leu Gly Leu
                   165                 170                 175
   Asp Lys Glu Cys Ile Lys Lys Val Leu Gly Asp Gln Gly Gln His Met
               180                 185                 190
   Ala Val Asn Tyr Tyr Pro Pro Cys Pro Gln Pro Asp Leu Thr Tyr Gly
           195                 200                 205
   Leu Pro Gly His Thr Asp Pro Asn Ala Leu Thr Ile Leu Leu Gln Asp
       210                 215                 220
   Leu Asn Val Ala Gly Leu Gln Val Leu Arg Asp Gly Arg Trp Leu Ala
   225                 230                 235                 240
   Val Lys Pro His Pro Asp Ala Phe Val Val Asn Ile Gly Asp Gln Leu
                   245                 250                 255
   Gln Ala Leu Ser Asn Gly Ile Tyr Lys Ser Val Trp His Arg Ala Val
               260                 265                 270
   Val Asn Ala Asp Gln Pro Arg Leu Ser Val Ala Ser Phe Leu Cys Pro
           275                 280                 285
   Cys Asp His Ala Val Ile Ser Ala Pro Lys Pro Leu Thr Ala Asp Gly
       290                 295                 300
   Ser Pro Val Val Tyr Arg Asp Phe Thr Tyr Ala Gln Tyr Tyr Lys Lys
   305                 310                 315                 320
   Phe Trp Ser Arg Asn Leu Asp Gln Glu His Cys Leu Glu Leu Phe Lys
                   325                 330                 335
   Asn
<210> 66
   <211> 1029
   <212> DNA
   <213> Cucumis sativus
<400> 66
   atgagcagtg tgatggagat ccaacttttg tgttcagggg gacgtcacga gaagttgcca      60
   gagaagtatg aacggcctga atcggatagg ccgcggctgt cggaggtgtg ttgttgggac     120
   aaggttccaa taatcgactt gggatgcgag gagagagaga tgattgtgaa gcaagtggag     180
   gaggcctgca agtcttacgg ctttttccag gttataaatc atggtgtgag gaaggaattg     240
   gtggagaaag tgatagaagt tggcaagcag ttctttgagc tgccgatgga ggagaagttg     300
   aaattttatt cagacgaccc ttccaagacc gtcagactct ccacaagttt caatgtccgg     360
   aaagagcaat ttcgcaactg gagggattat ctcagactcc attgctatcc tctctccaac     420
   tacacccccc attggccctc taacccacca tccttcaggg aaatagtgag tagttattgc     480
   aatgaagtac gaaaagttgg gtacagaata gaggagctaa tatcggagag cttggggctg     540
   gagaaggaat acataaggaa gaagttgggt gaacaaggtc agcacatggc tataaattat     600
   tatccgccat gtccccaacc agaactcacc tacgggctcc ctggccatac ggatcccaac     660
   gcactcacca ttctccttca ggatctccat gtcgccggcc tccaagtcct caaagatgga     720
   aagtggctag cggtcaaccc ccaccccaat gcctttgtaa tcaatatagg cgaccaattg     780
   caggcattga gcaatggggt gtacaagagc gtttggcacc gagcggtggt caatgttgat     840
   aagcccaggc tgtcggtcgc ttcttttctc tgcccttgtg atgacgccct cattactcct     900
   gcaccgctcc tctcccagcc ttcccccatt tacagacctt tcacctacgc ccagtactac     960
   aatacttttt ggagcagaaa cttggatcaa caacattgct tggaactatt taaaaaccac    1020
   cctccttaa                                                            1029
<210> 67
   <211> 342
   <212> PRT
   <213> Cucumis sativus
<400> 67
   Met Ser Ser Val Met Glu Ile Gln Leu Leu Cys Ser Gly Gly Arg His
   1               5                   10                  15
   Glu Lys Leu Pro Glu Lys Tyr Glu Arg Pro Glu Ser Asp Arg Pro Arg
               20                  25                  30
   Leu Ser Glu Val Cys Cys Trp Asp Lys Val Pro Ile Ile Asp Leu Gly
           35                  40                  45
   Cys Glu Glu Arg Glu Met Ile Val Lys Gln Val Glu Glu Ala Cys Lys
       50                  55                  60
   Ser Tyr Gly Phe Phe Gln Val Ile Asn His Gly Val Arg Lys Glu Leu
   65                  70                  75                  80
   Val Glu Lys Val Ile Glu Val Gly Lys Gln Phe Phe Glu Leu Pro Met
                   85                  90                  95
   Glu Glu Lys Leu Lys Phe Tyr Ser Asp Asp Pro Ser Lys Thr Val Arg
               100                 105                 110
   Leu Ser Thr Ser Phe Asn Val Arg Lys Glu Gln Phe Arg Asn Trp Arg
           115                 120                 125
   Asp Tyr Leu Arg Leu His Cys Tyr Pro Leu Ser Asn Tyr Thr Pro His
       130                 135                 140
   Trp Pro Ser Asn Pro Pro Ser Phe Arg Glu Ile Val Ser Ser Tyr Cys
   145                 150                 155                 160
   Asn Glu Val Arg Lys Val Gly Tyr Arg Ile Glu Glu Leu Ile Ser Glu
                   165                 170                 175
   Ser Leu Gly Leu Glu Lys Glu Tyr Ile Arg Lys Lys Leu Gly Glu Gln
               180                 185                 190
   Gly Gln His Met Ala Ile Asn Tyr Tyr Pro Pro Cys Pro Gln Pro Glu
               195                 200                 205
   Leu Thr Tyr Gly Leu Pro Gly His Thr Asp Pro Asn Ala Leu Thr Ile
       210                 215                 220
   Leu Leu Gln Asp Leu His Val Ala Gly Leu Gln Val Leu Lys Asp Gly
   225                 230                 235                 240
   Lys Trp Leu Ala Val Asn Pro His Pro Asn Ala Phe Val Ile Asn Ile
                   245                 250                 255
   Gly Asp Gln Leu Gln Ala Leu Ser Asn Gly Val Tyr Lys Ser Val Trp
               260                 265                 270
   His Arg Ala Val Val Asn Val Asp Lys Pro Arg Leu Ser Val Ala Ser
           275                 280                 285
   Phe Leu Cys Pro Cys Asp Asp Ala Leu Ile Thr Pro Ala Pro Leu Leu
       290                 295                 300
   Ser Gln Pro Ser Pro Ile Tyr Arg Pro Phe Thr Tyr Ala Gln Tyr Tyr
   305                 310                 315                 320
   Asn Thr Phe Trp Ser Arg Asn Leu Asp Gln Gln His Cys Leu Glu Leu
                   325                 330                 335
   Phe Lys Asn His Pro Pro
               340
<210> 68
   <211> 337
   <212> PRT
   <213> Gossypium hirsutum
<400> 68
   Met Asp Thr Lys Val Leu Ser Ser Gly Ile His Tyr Ser Ser Leu Pro
   1               5                   10                  15
   Glu Ser Tyr Val Arg Pro Glu Ser Glu Arg Pro Arg Leu Ser Glu Val
               20                  25                  30
   Ser Gln Cys Asp Asn Val Pro Val Ile Asp Leu Gly Cys Glu Asp Arg
           35                  40                  45
   Ser His Ile Val Gln Gln Ile Ala Leu Ala Cys Ile Asn Tyr Gly Phe
       50                  55                  60
   Phe Gln Val Ile Asn His Gly Val Ser Lys Glu Ala Val Glu Arg Met
   65                  70                  75                  80
   Leu Gln Val Ala His Asp Phe Phe Gly Leu Pro Val Glu Glu Lys Met
                   85                  90                  95
   Lys Leu Tyr Ser Asp Asp Pro Ser Lys Thr Met Arg Leu Ser Thr Ser
               100                 105                 110
   Phe Asn Val Lys Lys Glu Lys Val His Asn Trp Arg Asp Tyr Leu Arg
           115                 120                 125
   Leu His Cys Tyr Pro Leu His Lys Tyr Val Pro Glu Trp Pro Ser Asn
       130                 135                 140
   Pro Pro Ser Phe Lys Gln Ile Val Ser Asp Tyr Cys Val Gln Val Arg
   145                 150                 155                 160
   Glu Leu Gly Tyr Arg Leu Gln Glu Leu Ile Ser Glu Ser Leu Gly Leu
                   165                 170                 175
   Glu Lys Asp Tyr Ile Lys Lys Val Leu Gly Glu Gln Gly Gln His Met
               180                 185                 190
   Ala Val Asn Tyr Tyr Pro Pro Cys Pro Glu Pro Glu Leu Thr Tyr Gly
           195                 200                 205
   Leu Pro Gly His Thr Asp Pro Asn Ala Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Asn Pro Gln Thr Asn Ala Phe Val Ile Asn Ile Gly Asp Gln Leu
                   245                 250                 255
   Gln Ala Leu Ser Asn Gly Thr Tyr Lys Ser Val Trp His Arg Ala Ile
               260                 265                 270
   Val Asn Thr Asp Lys Pro Arg Met Ser Val Ala Ser Phe Leu Cys Pro
           275                 280                 285
   Tyr Asp His Ala Leu Ile Ser Pro Ala Lys Pro Leu Thr Gln His Gly
       290                 295                 300
   Cys Gly Ala Val Tyr Arg Asp Phe Thr Tyr Ala Glu Tyr Tyr Ser Lys
   305                 310                 315                 320
   Phe Trp Gly Arg Asn Leu Asp Gln Glu His Cys Leu Glu Leu Phe Lys
                   325                 330                 335
   Asn
<210> 69
   <211> 1014
   <212> DNA
   <213> Lactuca sativa
<400> 69
   atggccgcaa aagtcatctc cagtggattc cggtatacta ctctaccgga gagctacgtc      60
   cgtccggtta acgacagacc taacctatct caagtttccg attgcaacga cgttcctgtt     120
   attgacatcg gttgtggtga tagacaactc ataagccaac aaattggcga tgcttgtaga     180
   agatacggtt ttttccaggt gattaatcat ggtgtgcctg atgaaatagt ggagaaaatg     240
   caacaagtag gtagggagtt tttcctgttg cctgtggaag agaagatgaa gctttactca     300
   gaggatccat cgaagacgat gaggctatcc accagcttta acgtccaaaa agaacaaatt     360
   cataactggc gagattatct ccgccttcac tgttatcctc tggatcaata cagtcctgaa     420
   tggccttcaa atccttctta tttcaaggaa tatgttggta attattgtac agcagtgcga     480
   aatttaggaa tgagaatatt agaatcaata tcagaaagtt tagggttaca aaaagaagaa     540
   ataaaaacta tattaggcga tcaaggtcaa cacatggcca tcaaccatta cccagtgtgc     600
   cctgagcccg agctaaccta cgggctaccc gggcacacag accccaatgc tctcaccatc     660
   cttctacagg acacactggt ctctggtctt caggttctca aagatggcaa atggttagcc     720
   gttaaaccac accctaatgc gtttgtaatt aacattggtg atcagttaga ggcggtgagt     780
   aatggtgaat ataaaagtgt atggcatcga gctgtggtta actcagacaa cccgcgaatg     840
   tctatagctt cgtttttgtg tccttgtaat gacaccgtta ttagggctcc taaagaaata     900
   ataaaggaag gatcgaaacc tgttttcaaa gaatttactt atgcagaata ctacgcgaag     960
   ttttggacaa gaaaccttga tcaagaacat tgcttagaat tcttcaagaa ctag          1014
<210> 70
   <211> 337
   <212> PRT
   <213> Lactuca sativa
<400> 70
   Met Ala Ala Lys Val Ile Ser Ser Gly Phe Arg Tyr Thr Thr Leu Pro
   1               5                   10                  15
   Glu Ser Tyr Val Arg Pro Val Asn Asp Arg Pro Asn Leu Ser Gln Val
               20                  25                  30
   Ser Asp Cys Asn Asp Val Pro Val Ile Asp Ile Gly Cys Gly Asp Arg
           35                  40                  45
   Gln Leu Ile Ser Gln Gln Ile Gly Asp Ala Cys Arg Arg Tyr Gly Phe
           50                  55                  60
   Phe Gln Val Ile Asn His Gly Val Pro Asp Glu Ile Val Glu Lys Met
   65                  70                  75                  80
   Gln Gln Val Gly Arg Glu Phe Phe Leu Leu Pro Val Glu Glu Lys Met
                   85                  90                  95
   Lys Leu Tyr Ser Glu Asp Pro Ser Lys Thr Met Arg Leu Ser Thr Ser
               100                 105                 110
   Phe Asn Val Gln Lys Glu Gln Ile His Asn Trp Arg Asp Tyr Leu Arg
           115                 120                 125
   Leu His Cys Tyr Pro Leu Asp Gln Tyr Ser Pro Glu Trp Pro Ser Asn
       130                 135                 140
   Pro Ser Tyr Phe Lys Glu Tyr Val Gly Asn Tyr Cys Thr Ala Val Arg
   145                 150                 155                 160
   Asn Leu Gly Met Arg Ile Leu Glu Ser Ile Ser Glu Ser Leu Gly Leu
                   165                 170                 175
   Gln Lys Glu Glu Ile Lys Thr Ile Leu Gly Asp Gln Gly Gln His Met
               180                 185                 190
   Ala Ile Asn His Tyr Pro Val Cys Pro Glu Pro Glu Leu Thr Tyr Gly
           195                 200                 205
   Leu Pro Gly His Thr Asp Pro Asn Ala Leu Thr Ile Leu Leu Gln Asp
       210                 215                 220
   Thr Leu Val Ser Gly Leu Gln Val Leu Lys Asp Gly Lys Trp Leu Ala
   225                 230                 235                 240
   Val Lys Pro His Pro Asn Ala Phe Val Ile Asn Ile Gly Asp Gln Leu
                   245                 250                 255
   Glu Ala Val Ser Asn Gly Glu Tyr Lys Ser Val Trp His Arg Ala Val
               260                 265                 270
   Val Asn Ser Asp Asn Pro Arg Met Ser Ile Ala Ser Phe Leu Cys Pro
           275                 280                 285
   Cys Asn Asp Thr Val Ile Arg Ala Pro Lys Glu Ile Ile Lys Glu Gly
       290                 295                 300
   Ser Lys Pro Val Phe Lys Glu Phe Thr Tyr Ala Glu Tyr Tyr Ala Lys
   305                 310                 315                 320
   Phe Trp Thr Arg Asn Leu Asp Gln Glu His Cys Leu Glu Phe Phe Lys
   325 330 335
   Asn
<210> 71
   <211> 338
   <212> PRT
   <213> Medicago truncatula
<400> 71
   Met Asp Thr Lys Val Leu Ser Ser Gly Ile His Tyr Ser Lys Leu Pro
   1 5 10 15
   Glu Ser Tyr Ile Arg Pro Glu Ser Asp Arg Pro Cys Leu Ser Gln Val
   20 25 30
   Ser Glu Phe Glu Asn Val Pro Ile Ile Asp Leu Gly Ser His Asn Arg
   35 40 45
   Thr Gln Ile Val Gln Gln Ile Gly Glu Ala Cys Ser Ser Tyr Gly Phe
   50 55 60
   Phe Gln Val Val Asn His Gly Val Pro Leu Glu Glu Leu Lys Lys Thr
   65 70 75 80
   Ala Glu Val Ala Tyr Asp Phe Phe Lys Leu Pro Val Glu Glu Lys Met
   85 90 95
   Lys Leu Tyr Ser Asp Asp Pro Thr Lys Thr Met Arg Leu Ser Thr Ser
   100 105 110
   Phe Asn Val Asn Lys Glu Glu Val His Asn Trp Arg Asp Tyr Leu Arg
   115 120 125
   Leu His Cys Tyr Pro Leu Asp Asn Tyr Val Pro Glu Trp Pro Ser Asn
   130 135 140
   Pro Pro Ser Phe Lys Glu Thr Val Ala Asn Tyr Cys Lys Glu Val Arg
   145 150 155 160
   Glu Leu Gly Leu Arg Ile Glu Glu Tyr Ile Ser Glu Ser Leu Gly Leu
   165 170 175
   Glu Lys Asp Tyr Leu Arg Asn Ala Leu Gly Glu Gln Gly Gln His Met
   180 185 190
   Ala Val Asn Tyr Tyr Pro Pro Cys Pro Gln Pro Glu Leu Thr Tyr Gly
   195 200 205
   Leu Pro Gly His Thr Asp Pro Asn Ala Leu Thr Ile Leu Leu Gln Asp
   210 215 220
   Leu His Val Ala Gly Leu Gln Val Leu Lys Asp Gly Lys Trp Leu Ala
   225 230 235 240
   Ile Asn Pro Ile Pro Asp Ala Phe Val Ile Asn Ile Gly Asp Gln Leu
   245 250 255
   Gln Ala Leu Ser Asn Gly Leu Tyr Lys Ser Val Trp His Arg Ala Ile
   260 265 270
   Val Asn Ala Glu Lys Pro Arg Leu Ser Val Ala Ser Phe Leu Cys Pro
   275 280 285
   Asp Asn Glu Ala Leu Ile Cys Pro Ala Lys Pro Leu Thr Glu Asp Gly
   290 295 300
   Ser Gly Ala Val Tyr Arg Gly Phe Thr Tyr Pro Glu Tyr Tyr Ser Lys
   305 310 315 320
   Phe Trp Ser Arg Asp Leu Glu Lys Glu His Cys Leu Glu Phe Phe Lys
   325 330 335
   Asn Asn
<210> 72
   <211> 342
   <212> PRT
   <213> Oryza sativa
<400> 72
   Met Ala Ala Glu Ala Glu Gln Gln His Gln Leu Leu Ser Thr Ala Val
   1               5                   10                  15
   His Asp Thr Met Pro Gly Lys Tyr Val Arg Pro Glu Ser Gln Arg Pro
               20                  25                  30
   Arg Leu Asp Leu Val Val Ser Asp Ala Arg Ile Pro Val Val Asp Leu
           35                  40                  45
   Ala Ser Pro Asp Arg Ala Ala Val Val Ser Ala Val Gly Asp Ala Cys
       50                  55                  60
   Arg Thr His Gly Phe Phe Gln Val Val Asn His Gly Ile Asp Ala Ala
   65                  70                  75                  80
   Leu Ile Ala Ser Val Met Glu Val Gly Arg Glu Phe Phe Arg Leu Pro
                   85                  90                  95
   Ala Glu Glu Lys Ala Lys Leu Tyr Ser Asp Asp Pro Ala Lys Lys Ile
               100                 105                 110
   Arg Leu Ser Thr Ser Phe Asn Val Arg Lys Glu Thr Val His Asn Trp
           115                 120                 125
   Arg Asp Tyr Leu Arg Leu His Cys Tyr Pro Leu His Gln Phe Val Pro
       130                 135                 140
   Asp Trp Pro Ser Asn Pro Pro Ser Phe Lys Glu Ile Ile Gly Thr Tyr
   145                 150                 155                 160
   Cys Thr Glu Val Arg Glu Leu Gly Phe Arg Leu Tyr Glu Ala Ile Ser
                   165                 170                 175
   Glu Ser Leu Gly Leu Glu Gly Gly Tyr Met Arg Glu Thr Leu Gly Glu
               180                 185                 190
   Gln Glu Gln His Met Ala Val Asn Tyr Tyr Pro Gln Cys Pro Glu Pro
           195                 200                 205
   Glu Leu Thr Tyr Gly Leu Pro Ala His Thr Asp Pro Asn Ala Leu Thr
       210                 215                 220
   Ile Leu Leu Met Asp Asp Gln Val Ala Gly Leu Gln Val Leu Asn Asp
   225                 230                 235                 240
   Gly Lys Trp Ile Ala Val Asn Pro Gln Pro Gly Ala Leu Val Ile Asn
                   245                 250                 255
   Ile Gly Asp Gln Leu Gln Ala Leu Ser Asn Gly Lys Tyr Arg Ser Val
               260                 265                 270
   Trp His Arg Ala Val Val Asn Ser Asp Arg Glu Arg Met Ser Val Ala
           275                 280                 285
   Ser Phe Leu Cys Pro Cys Asn Ser Val Glu Leu Gly Pro Ala Lys Lys
       290                 295                 300
   Leu Ile Thr Asp Asp Ser Pro Ala Val Tyr Arg Asn Tyr Thr Tyr Asp
   305                 310                 315                 320
   Glu Tyr Tyr Lys Lys Phe Trp Ser Arg Asn Leu Asp Gln Glu His Cys
                   325                 330                 335
   Leu Glu Leu Phe Arg Thr
   340
<210> 73
   <211> 342
   <212> PRT
   <213> Oryza sativa
<400> 73
   Met Ala Asp Gln Leu Ile Ser Thr Ala Asp His Asp Thr Leu Pro Gly
   1               5                   10                  15
   Asn Tyr Val Arg Pro Glu Ala Gln Arg Pro Arg Leu Ala Asp Val Leu
               20                  25                  30
   Ser Asp Ala Ser Ile Pro Val Val Asp Leu Ala Asn Pro Asp Arg Ala
           35                  40                  45
   Lys Leu Val Ser Gln Val Gly Ala Ala Cys Arg Ser His Gly Phe Phe
       50                  55                  60
   Gln Val Leu Asn His Gly Val Pro Val Glu Leu Thr Leu Ser Val Leu
   65                  70                  75                  80
   Ala Val Ala His Asp Phe Phe Arg Leu Pro Ala Glu Glu Lys Ala Lys
                   85                  90                  95
   Leu Tyr Ser Asp Asp Pro Ala Lys Lys Ile Arg Leu Ser Thr Ser Phe
               100                 105                 110
   Asn Val Arg Lys Glu Thr Val His Asn Trp Arg Asp Tyr Leu Arg Leu
           115                 120                 125
   His Cys Tyr Pro Leu His Arg Tyr Leu Pro Asp Trp Pro Ser Asn Pro
       130                 135                 140
   Pro Ser Phe Arg Glu Ile Ile Ser Thr Tyr Cys Lys Glu Val Arg Glu
   145                 150                 155                 160
   Leu Gly Phe Arg Leu Tyr Gly Ala Ile Ser Glu Ser Leu Gly Leu Glu
                   165                 170                 175
   Gln Asp Tyr Ile Lys Lys Val Leu Gly Glu Gln Glu Gln His Met Ala
               180                 185                 190
   Val Asn Phe Tyr Pro Lys Cys Pro Glu Pro Glu Leu Thr Phe Gly Leu
           195                 200                 205
   Pro Ala His Thr Asp Pro Asn Ala Leu Thr Ile Leu Leu Met Asp Gln
       210                 215                 220
   Gln Val Ala Gly Leu Gln Val Leu Lys Glu Gly Arg Trp Ile Ala Val
   225                 230                 235                 240
   Asn Pro Gln Pro Asn Ala Leu Val Ile Asn Ile Gly Asp Gln Leu Gln
                   245                 250                 255
   Ala Leu Ser Asn Gly Arg Tyr Lys Ser Val Trp His Arg Ala Val Val
               260                 265                 270
   Asn Ser Asp Lys Ala Arg Met Ser Val Ala Ser Phe Leu Cys Pro Cys
           275                 280                 285
   Asn Asp Val Leu Ile Gly Pro Ala Gln Lys Leu Ile Thr Asp Gly Ser
       290                 295                 300
   Pro Ala Val Tyr Arg Asn Tyr Thr Tyr Asp Glu Tyr Tyr Lys Lys Phe
   305                 310                 315                 320
   Trp Ser Arg Asn Leu Asp Gln Glu His Cys Leu Glu Leu Phe Arg Thr
                   325                 330                 335
   Thr Pro Thr Asp Thr Ser
               340
<210> 74
   <211> 340
   <212> PRT
   <213> Oryza sativa
<400> 74
   Met Ala Thr Thr Gln Leu Leu Ser Thr Val Glu His Arg Glu Thr Leu
   1               5                   10                  15
   Pro Glu Gly Tyr Ala Arg Pro Glu Ser Asp Arg Pro Arg Leu Ala Glu
               20                  25                  30
   Val Ala Thr Asp Ser Asn Ile Pro Leu Ile Asp Leu Ala Ser Pro Asp
           35                  40                  45
   Lys Pro Arg Val Ile Ala Glu Ile Ala Gln Ala Cys Arg Thr Tyr Gly
       50                  55                  60
   Phe Phe Gln Val Thr Asn His Gly Ile Ala Glu Glu Leu Leu Glu Lys
   65                  70                  75                      80
   Val Met Ala Val Ala Leu Glu Phe Phe Arg Leu Pro Pro Glu Glu Lys
                   85                  90                  95
   Glu Lys Leu Tyr Ser Asp Glu Pro Ser Lys Lys Ile Arg Leu Ser Thr
               100                 105                 110
   Ser Phe Asn Val Arg Lys Glu Thr Val His Asn Trp Arg Asp Tyr Leu
           115                 120                 125
   Arg Leu His Cys His Pro Leu Glu Glu Phe Val Pro Glu Trp Pro Ser
       130                 135                 140
   Asn Pro Ala Gln Phe Lys Glu Ile Met Ser Thr Tyr Cys Arg Glu Val
   145                 150                 155                 160
   Arg Gln Leu Gly Leu Arg Leu Leu Gly Ala Ile Ser Val Ser Leu Gly
                   165                 170                 175
   Leu Glu Glu Asp Tyr Ile Glu Lys Val Leu Gly Glu Gln Glu Gln His
               180                 185                 190
   Met Ala Val Asn Tyr Tyr Pro Arg Cys Pro Glu Pro Asp Leu Thr Tyr
           195                 200                 205
   Gly Leu Pro Lys His Thr Asp Pro Asn Ala Leu Thr Ile Leu Leu Pro
       210                 215                 220
   Asp Pro His Val Ala Gly Leu Gln Val Leu Arg Asp Gly Asp Gln Trp
   225                 230                 235                 240
   Ile Val Val Asn Pro Arg Pro Asn Ala Leu Val Val Asn Leu Gly Asp
                   245                 250                 255
   Gln Ile Gln Ala Leu Ser Asn Asp Ala Tyr Lys Ser Val Trp His Arg
               260                 265                 270
   Ala Val Val Asn Pro Val Gln Glu Arg Met Ser Val Ala Ser Phe Met
           275                 280                 285
   Cys Pro Cys Asn Ser Ala Val Ile Ser Pro Ala Arg Lys Leu Val Ala
       290                 295                 300
   Asp Gly Asp Ala Pro Val Tyr Arg Ser Phe Thr Tyr Asp Glu Tyr Tyr
   305                 310                 315                 320
   Lys Lys Phe Trp Ser Arg Asn Leu Asp Gln Glu His Cys Leu Glu Leu
                   325                 330                 335
   Phe Lys Gly Gln
               340
<210> 75
   <211> 338
   <212> PRT
   <213> Populus trichocarpa
<400> 75
   Met Asp Thr Lys Val Leu Ser Ser Gly Ile Gln Tyr Thr Asn Leu Pro
   1               5                   10                  15
   Ala Ser Tyr Val Arg Pro Glu Ser Glu Arg Pro Arg Leu Trp Glu Val
               20                  25                  30
   Ser Thr Cys Glu Asn Val Pro Val Ile Asp Leu Gly Cys Gln Glu Arg
           35                  40                  45
   Asp Gln Ile Val Gln Gln Val Gly Asp Ala Cys Lys Asn Tyr Gly Phe
       50                  55                  60
   Phe Gln Val Ile Asn His Gly Val Ser Leu Glu Ala Val Glu Lys Met
   65                  70                  75                  80
   Leu Gly Val Ala His Asp Phe Phe Ser Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Asn Lys Glu Lys Val His Asn Trp Arg Asp Tyr Leu Arg
           115                 120                 125
   Leu His Cys Tyr Pro Leu Asp Lys Tyr Ala Pro Glu Trp Pro Ser Lys
       130                 135                 140
   Pro Pro Pro Phe Lys Asp Ile Val Ser Ser Tyr Cys Ile Gln Val Arg
   145                 150                 155                 160
   Glu Leu Gly Phe Arg Ile Gln Glu Leu Ile Ser Glu Ser Leu Gly Leu
                   165                 170                 175
   Glu Lys Asp His Val Lys Asn Val Leu Gly Glu Gln Gly Gln His Met
               180                 185                 190
   Ala Val Asn Phe Tyr Pro Pro Cys Pro Glu Pro Glu Leu Thr Phe Gly
           195                 200                 205
   Leu Pro Gly His Thr Asp Pro Asn Ala Leu Thr Ile Leu Leu Gln Asp
       210                 215                 220
   Gln Ser Val Ala Gly Leu Gln Val Leu Lys Asp Gly Lys Trp Val Ala
   225                 230                 235                 240
   Val Asp Pro His Pro Asp Ala Phe Val Ile Asn Ile Gly Asp Gln Leu
   245 250 255
   Gln Ala Leu Ser Asn Gly Arg Tyr Lys Ser Val Trp His Arg Ala Ile
   260 265 270
   Thr Asn Thr Asp Lys Ala Arg Met Ser Val Ala Ser Phe Leu Cys Pro
   275 280 285
   Tyr Asp Asn Ala Leu Ile Thr Pro Pro Lys Ala Leu Thr Asp Asp Gly
   290 295 300
   Thr Gly Ala Val Tyr Arg Asp Phe Thr Tyr Ala Glu Tyr Tyr Lys Lys
   305 310 315 320
   Phe Trp Ser Arg Asp Leu Asp Gln Glu His Cys Leu Glu Leu Phe Lys
   325 330 335
   Asn Lys
<210> 76
   <211> 338
   <212> PRT
   <213> Populus trichocarpa
<400> 76
   Met Asp Thr Lys Val Ile Ser Ser Gly Val His Tyr Thr Asn Leu Pro
   1 5 10 15
   Ala Ser Tyr Val Arg Pro Glu Ser Glu Arg Pro Arg Leu Ser Glu Val
   20 25 30
   Ser Thr Cys Glu Asp Val Pro Val Ile Asp Leu Gly Cys Gln Asp Arg
   35 40 45
   Asn Gln Ile Val Gln Gln Val Gly Asp Ala Cys Glu His Tyr Gly Phe
   50 55 60
   Phe Gln Val Ile Asn His Gly Val Ser Leu Glu Ala Val Glu Lys Met
   65 70 75 80
   Leu Gly Val Ala His Asp Phe Phe Ser Leu Pro Val Glu Glu Lys Leu
   85 90 95
   Lys Leu Tyr Ser Asp Asp Pro Ser Lys Thr Met Arg Leu Ser Thr Ser
   100 105 110
   Phe Asn Val Asn Lys Glu Lys Val His Asn Trp Arg Asp Tyr Leu Arg
   115 120 125
   Leu His Cys Tyr Pro Leu Asp Lys Tyr Val Pro Glu Trp Pro Ser Asn
   130 135 140
   Pro Pro Pro Phe Lys Glu Ile Val Arg Ser Tyr Ser Ile Gln Val Arg
   145 150 155 160
   Glu Leu Gly Phe Arg Ile Gln Glu Leu Ile Ser Glu Ser Leu Gly Leu
   165 170 175
   Glu Lys Asp His Ile Lys Asn Val Leu Gly Glu Gln Gly Gln His Met
   180 185 190
   Ala Val Asn Phe Tyr Pro Pro Cys Pro Glu Pro Glu Leu Thr Tyr Gly
   195 200 205
   Leu Pro Ala His Thr Asp Pro Asn Ala Leu Thr Ile Leu Leu Gln Asp
   210 215 220
   Leu Ser Val Ala Gly Leu Gln Val Leu Leu Lys Asp Gly Lys Trp Val
   225 230 235 240
   Ala Val Asn Pro His Pro Asp Ala Phe Val Ile Asn Ile Gly Asp Gln
   245 250 255
   Leu Gln Ala Leu Ser Asn Gly Arg Tyr Lys Ser Val Trp His Arg Ala
   260 265 270
   Ile Thr Asn Thr Asp Lys Ala Arg Met Ser Val Ala Ser Phe Leu Cys
   275 280 285
   Pro Phe Asp Asn Ala Leu Ile Thr Pro Pro Lys Ala Leu Thr Asp Asp
   290 295 300
   Gly Thr Gly Ala Ile Tyr Arg Asp Phe Thr Tyr Ala Glu Tyr Tyr Lys
   305 310 315 320
   Lys Phe Trp Ser Arg Asn Leu Asp Gln Glu His Cys Leu Glu Leu Phe
   325 330 335
   Lys Asn
<210> 77
   <211> 337
   <212> PRT
   <213> Solanum lycopersicum
<400> 77
   Met Glu Thr Lys Val Ile Ser Ser Gly Ile Asn His Ser Thr Leu Pro
   1 5 10 15
   Gln Ser Tyr Ile Arg Pro Glu Ser Asp Arg Pro Arg Leu Ser Glu Val
   20 25 30
   Val Asp Cys Glu Asn Val Pro Ile Ile Asp Leu Ser Cys Gly Asp Gln
   35 40 45
   Ala Gln Ile Ile Arg Gln Ile Gly Glu Ala Cys Gln Thr Tyr Gly Phe
   50 55 60
   Phe Gln Val Ile Asn His Gly Val Pro Lys Glu Val Val Glu Lys Met
   65 70 75 80
   Leu Gly Val Ala Gly Glu Phe Phe Asn Leu Pro Val Glu Glu Lys Leu
   85 90 95
   Lys Leu Tyr Ser Asp Asp Pro Ser Lys Thr Met Arg Leu Ser Thr Ser
   100 105 110
   Phe Asn Val Lys Lys Glu Thr Val His Asn Trp Arg Asp Tyr Leu Arg
   115 120 125
   Leu His Cys Tyr Pro Leu Glu Lys Tyr Ala Pro Glu Trp Pro Ser Asn
   130 135 140
   Pro Ser Ser Phe Arg Glu Ile Val Ser Arg Tyr Cys Arg Glu Ile Arg
   145 150 155 160
   Gln Leu Gly Phe Arg Leu Glu Glu Ala Ile Ala Glu Ser Leu Gly Leu
   165 170 175
   Asp Lys Glu Cys Ile Lys Asp Val Leu Gly Glu Gln Gly Gln His Met
   180 185 190
   Ala Ile Asn Tyr Tyr Pro Pro Cys Pro Gln Pro Glu Leu Thr Tyr Gly
   195 200 205
   Leu Pro Ala His Thr Asp Pro Asn Ser Leu Thr Ile Leu Leu Gln Asp
   210 215 220
   Leu Gln Val Ala Gly Leu Gln Val Leu Lys Asp Gly Lys Trp Leu Ala
   225 230 235 240
   Val Lys Pro Gln Pro Asp Ala Phe Val Ile Asn Leu Gly Asp Gln Leu
   245 250 255
   Gln Ala Val Ser Asn Gly Lys Tyr Arg Ser Val Trp His Arg Ala Ile
               260                 265                 270
   Val Asn Ser Asp Gln Ala Arg Met Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Asn Lys
   305                 310                 315                 320
   Phe Trp Ser Arg Asn Leu Asp Gln Gln His Cys Leu Glu Leu Phe Lys
                   325                 330                 335
   Asn
<210> 78
   <211> 342
   <212> PRT
   <213> Solanum lycopersicum
<400> 78
   Met Thr Thr Thr Ser Val Leu Ser Ser Gly Phe Asn His Ser Thr Leu
   1               5                   10                  15
   Pro Gln Ser Tyr Val Arg Pro Glu Ser Gln Arg Pro Cys Met Ser Glu
               20                  25                  30
   Val Val Asp Ser Asp Asp Leu Val Pro Val Ile Asp Met Ser Cys Thr
           35                  40                  45
   Asn Arg Asn Val Ile Val His Gln Ile Gly Glu Ala Cys Arg Leu Tyr
       50                  55                  60
   Gly Phe Phe Gln Val Ile Asn His Gly Val Ser Lys Lys Val Ile Asp
   65                  70                  75                  80
   Glu Met Leu Gly Val Ser His Glu Phe Phe Lys Leu Pro Val Glu Glu
                   85                  90                  95
   Lys Met Lys Leu Tyr Ser Asp Asp Pro Ser Lys Thr Met Arg Leu Ser
               100                 105                 110
   Thr Ser Phe Asn Val Lys Lys Glu Thr Val His Asn Trp Arg Asp Tyr
           115                 120                 125
   Leu Arg Leu His Cys Tyr Pro Leu Asp Lys Tyr Ala Pro Glu Trp Pro
   130 135 140
   Ser Asn Pro Pro Ser Phe Arg Glu Ile Val Ser Lys Tyr Cys Met Glu
   145 150 155 160
   Val Arg Glu Leu Gly Tyr Arg Leu Glu Glu Ala Ile Ser Glu Ser Leu
   165 170 175
   Gly Leu Glu Lys Asp Cys Ile Lys Asn Val Leu Gly Glu Gln Gly Gln
   180 185 190
   His Met Ala Ile Asn Phe Tyr Pro Gln Cys Pro Gln Pro Glu Leu Thr
   195 200 205
   Tyr Gly Leu Pro Ala His Thr Asp Pro Asn Ala Ile Thr Ile Leu Leu
   210 215 220
   Gln Asp Leu Gln Val Ala Gly Leu Gln Val Leu Lys Asp Gly Lys Trp
   225 230 235 240
   Leu Ser Ile Lys Pro Gln Pro Asn Ala Phe Val Ile Asn Leu Gly Asp
   245 250 255
   Gln Leu Glu Ala Leu Ser Asn Gly Lys Tyr Lys Ser Ile Trp His Arg
   260 265 270
   Ala Ile Val Asn Ser Asp Lys Ala Arg Met Ser Val Ala Ser Phe Leu
   275 280 285
   Cys Pro Asn Asp Cys Ser Ile Ile Ser Ala Pro Lys Thr Leu Thr Glu
   290 295 300
   Asp Gly Ser Ser Ala Ile Tyr Arg His Phe Thr Tyr Ala Glu Tyr Tyr
   305 310 315 320
   Glu Lys Phe Trp Ser Arg Asn Leu Asp Gln Glu Tyr Cys Leu Glu Leu
   325 330 335
   Phe Lys Asn Asp Gly Thr
   340
<210> 79
   <211> 336
   <212> PRT
   <213> Sorghum bicolor
<400> 79
   Met Ala Glu Gln Leu Leu Ser Thr Ala Val His Asp Thr Leu Pro Gly
   1               5                   10                  15
   Ser Tyr Val Arg Pro Glu Ser Gln Arg Pro Arg Leu Ala Glu Val Val
               20                  25                  30
   Thr Gly Ala Arg Ile Pro Val Val Asp Leu Gly Ser Pro Asp Arg Ala
           35                  40                  45
   Ala Val Val Ala Ala Ile Gly Asp Ala Cys Arg Ser His Gly Phe Phe
       50                  55                  60
   Gln Val Leu Asn His Gly Val His Ala Asp Leu Val Ala Ala Val Met
   65                  70                  75                  80
   Ala Val Gly Arg Ala Phe Phe Arg Leu Ser Pro Glu Glu Lys Ala Lys
                   85                  90                  95
   Leu Tyr Ser Asp Asp Pro Ala Arg Lys Ile Arg Leu Ser Thr Ser Phe
               100                 105                 110
   Asn Val Arg Lys Glu Thr Val His Asn Trp Arg Asp Tyr Leu Arg Leu
           115                 120                 125
   His Cys His Pro Leu Asp Glu Phe Val Pro Asp Trp Pro Ser Asn Pro
       130                 135                 140
   Pro Asp Phe Lys Asp Thr Met Ser Thr Tyr Cys Lys Glu Val Arg Glu
   145                 150                 155                 160
   Leu Gly Phe Arg Leu Tyr Ala Ala Ile Ser Glu Ser Leu Gly Leu Glu
                   165                 170                 175
   Ala Ser Tyr Met Lys Glu Thr Leu Gly Glu Gln Glu Gln His Met Ala
               180                 185                 190
   Val Asn Phe Tyr Pro Pro Cys Pro Glu Pro Glu Leu Thr Tyr Gly Leu
           195                 200                 205
   Pro Ala His Thr Asp Pro Asn Ala Leu Thr Ile Leu Leu Met Asp Gln
       210                 215                 220
   Asp Val Ala Gly Leu Gln Val Leu His Gly Gly Lys Trp Val Ala Val
   225                 230                 235                 240
   Asn Pro Gln Pro Gly Ala Leu Ile Ile Asn Ile Gly Asp Gln Leu Gln
                   245                 250                 255
   Ala Leu Ser Asn Gly Gln Tyr Arg Ser Val Trp His Arg Ala Val Val
               260                 265                 270
   Asn Ser Asp Arg Glu Arg Met Ser Val Ala Ser Phe Leu Cys Pro Cys
           275                 280                 285
   Asn His Val Val Leu Gly Pro Ala Lys Lys Leu Val Thr Glu Asp Thr
       290                 295                 300
   Pro Ala Val Tyr Arg Ser Tyr Thr Tyr Asp Glu Tyr Tyr Lys Lys Phe
   305                 310                 315                 320
   Trp Ser Arg Asn Leu Asp Gln Glu His Cys Leu Glu Leu Phe Arg Thr
                   325                 330                 335
<210> 80
   <211> 1020
   <212> DNA
   <213> Spinacia oleracea
<400> 80
   atggcaaaca agatattatc caccggaatt ccttacaaaa ccctccccga aagctacatc     60
   cgacccgaaa atgagaggcc caacttatct caagtctccg attgcgagaa tgtccctgtt    120
   attgacttgg gtgccaaaga ccgtactcaa acaatccacc aagtcttcaa tgcttgtaaa    180
   aattacgggt ttttccaggt gattaatcat ggggtgtcaa aggaattagc ggagaagatg    240
   caaaaggtag ctcgagagtt cttcgatatg tcggttgagg aaaaaatgaa attatatagt    300
   gacgatccaa ctaaaacact aagattgtct acaagtttta acgttaacaa agaggaagtt    360
   cataattgga gagattatct taggctccat tgttggcctc ttgagcaata tgtccccgaa    420
   tggccttcta accccccttc cttcaaggaa atagtgagca agtacataaa agaagttagg    480
   gaacttggtt tcagagtcca agaactaata tcagagagtt tagggttgga gaaagattac    540
   ataaagaatg tcctaggaga tcaaggacaa cacatggctc ttaattatta ccctgagtgc    600
   ccggagccag agatgacata cgggttgccg ggtcatactg accctaatgc ccttaccatc    660
   cttctccaag acttgcaagt atctggcctt caaattttta aggatggtaa atggcttgct    720
   gtcaaacctc aacctgatgc ttttgtcatt aacattggtg atcaattgca ggcattaagt    780
   aacggtatat acaagagtgt atggcacaga gcagttgtga acacagataa gccaagatta    840
   tcagtagctt cattcctctg ccccgccaat gatgcgttga taagcgcgcc aacacctctg    900
   accgccaacg gatcaccggc tgtatataga gactatacgt atcctgagta ctacaagact    960
   ttctggagta ggaacttgga ccaagagcac tgcttggagc tttttaaaaa ccaaacctag   1020
<210> 81
   <211> 339
   <212> PRT
   <213> Spinacia oleracea
<400> 81
   Met Ala Asn Lys Ile Leu Ser Thr Gly Ile Pro Tyr Lys Thr Leu Pro
   1               5                   10                  15
   Glu Ser Tyr Ile Arg Pro Glu Asn Glu Arg Pro Asn Leu Ser Gln Val
               20                  25                  30
   Ser Asp Cys Glu Asn Val Pro Val Ile Asp Leu Gly Ala Lys Asp Arg
           35                  40                  45
   Thr Gln Thr Ile His Gln Val Phe Asn Ala Cys Lys Asn Tyr Gly Phe
       50                  55                  60
   Phe Gln Val Ile Asn His Gly Val Ser Lys Glu Leu Ala Glu Lys Met
   65                  70                  75                  80
   Gln Lys Val Ala Arg Glu Phe Phe Asp Met Ser Val Glu Glu Lys Met
                   85                  90                  95
   Lys Leu Tyr Ser Asp Asp Pro Thr Lys Thr Leu Arg Leu Ser Thr Ser
               100                 105                 110
   Phe Asn Val Asn Lys Glu Glu Val His Asn Trp Arg Asp Tyr Leu Arg
           115                 120                 125
   Leu His Cys Trp Pro Leu Glu Gln Tyr Val Pro Glu Trp Pro Ser Asn
       130                 135                 140
   Pro Pro Ser Phe Lys Glu Ile Val Ser Lys Tyr Ile Lys Glu Val Arg
   145                 150                 155                 160
   Glu Leu Gly Phe Arg Val Gln Glu Leu Ile Ser Glu Ser Leu Gly Leu
                   165                 170                 175
   Glu Lys Asp Tyr Ile Lys Asn Val Leu Gly Asp Gln Gly Gln His Met
               180                 185                 190
   Ala Leu Asn Tyr Tyr Pro Glu Cys Pro Glu Pro Glu Met Thr Tyr Gly
           195                 200                 205
   Leu Pro Gly His Thr Asp Pro Asn Ala Leu Thr Ile Leu Leu Gln Asp
       210                 215                 220
   Leu Gln Val Ser Gly Leu Gln Ile Phe Lys Asp Gly Lys Trp Leu Ala
   225                 230                 235                 240
   Val Lys Pro Gln Pro Asp Ala Phe Val Ile Asn Ile Gly Asp Gln Leu
                   245                 250                 255
   Gln Ala Leu Ser Asn Gly Ile Tyr Lys Ser Val Trp His Arg Ala Val
               260                 265                 270
   Val Asn Thr Asp Lys Pro Arg Leu Ser Val Ala Ser Phe Leu Cys Pro
   275 280 285
   Ala Asn Asp Ala Leu Ile Ser Ala Pro Thr Pro Leu Thr Ala Asn Gly
   290 295 300
   Ser Pro Ala Val Tyr Arg Asp Tyr Thr Tyr Pro Glu Tyr Tyr Lys Thr
   305 310 315 320
   Phe Trp Ser Arg Asn Leu Asp Gln Glu His Cys Leu Glu Leu Phe Lys
   325 330 335
   Asn Gln Thr
<210> 82
   <211> 338
   <212> PRT
   <213> Vitis sp.
<400> 82
   Met Glu Ser Lys Val Leu Ser Thr Gly Ile Arg Tyr Leu Thr Leu Pro
   1 5 10 15
   Gln Ser Tyr Ile Arg Pro Glu Pro Glu Arg Pro Arg Leu Ser Gln Val
   20 25 30
   Ser Glu Cys Lys His Val Pro Ile Ile Asp Leu Gly Lys Asp Val Asn
   35 40 45
   Arg Ala Gln Leu Ile Gln His Ile Ala Asp Ala Cys Arg Leu Tyr Gly
   50 55 60
   Phe Phe Gln Val Ile Asn His Gly Val Ala Ala Glu Met Met Glu Lys
   65 70 75 80
   Met Leu Glu Val Ala Asp Glu Phe Tyr Arg Leu Pro Val Glu Glu Lys
   85 90 95
   Met Lys Leu Tyr Ser Asp Asp Pro Thr Lys Thr Met Arg Leu Ser Thr
   100 105 110
   Ser Phe Asn Val Asn Lys Glu Lys Val His Asn Trp Arg Asp Tyr Leu
   115 120 125
   Arg Leu His Cys Tyr Pro Leu Asp Gln Tyr Thr Pro Glu Trp Pro Ser
   130 135 140
   Asn Pro Pro Ser Phe Lys Glu Ile Val Ser Ser Tyr Cys Lys Glu Val
   145                 150                 155                 160
   Arg Glu Leu Gly Phe Arg Leu Gln Glu Met Ile Ser Glu Ser Leu Gly
                   165                 170                 175
   Leu Glu Lys Asp His Ile Lys Asn Val Phe Gly Glu Gln Gly Gln His
               180                 185                 190
   Met Ala Val Asn Tyr Tyr Pro Pro Cys Pro Gln Pro Glu Leu Thr Tyr
           195                 200                 205
   Gly Leu Pro Gly His Thr Asp Pro Asn Ala Leu Thr Ile Leu Leu Gln
       210                 215                 220
   Asp Leu Arg Val Ala Gly Leu Gln Val Leu Lys Asp Gly Thr Trp Leu
   225                 230                 235                 240
   Ala Ile Lys Pro His Pro Gly Ala Phe Val Val Asn Ile Gly Asp Gln
                   245                 250                 255
   Leu Gln Ala Val Ser Asn Gly Lys Tyr Lys Ser Val Trp His Arg Ala
               260                 265                 270
   Val Val Asn Ala Glu Ser Glu Arg Leu Ser Val Ala Ser Phe Leu Cys
           275                 280                 285
   Pro Cys Asn Asp Ala Val Ile Gly Pro Ala Lys Pro Leu Thr Glu Asp
       290                 295                 300
   Gly Ser Ala Pro Ile Tyr Lys Asn Phe Thr Tyr Ala Glu Tyr Tyr Lys
   305                 310                 315                 320
   Lys Phe Trp Gly Arg Asp Leu Asp Gln Glu His Cys Leu Glu Leu Phe
                   325                 330                 335
   Lys Asn
<210> 83
   <211> 336
   <212> PRT
   <213> Zea mays
<400> 83
   Met Ala Glu His Leu Leu Ser Thr Ala Val His Asp Thr Leu Pro Gly
   1               5                   10                  15
   Ser Tyr Val Arg Pro Glu Pro Glu Arg Pro Arg Leu Ala Glu Val Val
               20                  25                  30
   Thr Gly Ala Arg Ile Pro Val Val Asp Leu Gly Ser Pro Asp Arg Gly
           35                  40                  45
   Ala Val Val Ala Ala Val Gly Asp Ala Cys Arg Ser His Gly Phe Phe
       50                  55                  60
   Gln Val Val Asn His Gly Ile His Ala Ala Leu Val Ala Ala Val Met
   65                  70                  75                  80
   Ala Ala Gly Arg Gly Phe Phe Arg Leu Pro Pro Glu Glu Lys Ala Lys
                   85                  90                  95
   Leu Tyr Ser Asp Asp Pro Ala Arg Lys Ile Arg Leu Ser Thr Ser Phe
               100                 105                 110
   Asn Val Arg Lys Glu Thr Val His Asn Trp Arg Asp Tyr Leu Arg Leu
           115                 120                 125
   His Cys His Pro Leu Asp Glu Phe Leu Pro Asp Trp Pro Ser Asn Pro
       130                 135                 140
   Pro Asp Phe Lys Glu Thr Met Gly Thr Tyr Cys Lys Glu Val Arg Glu
   145                 150                 155                 160
   Leu Gly Phe Arg Leu Tyr Ala Ala Ile Ser Glu Ser Leu Gly Leu Glu
                   165                 170                 175
   Ala Ser Tyr Met Lys Glu Ala Leu Gly Glu Gln Glu Gln His Met Ala
               180                 185                 190
   Val Asn Phe Tyr Pro Pro Cys Pro Glu Pro Glu Leu Thr Tyr Gly Leu
           195                 200                 205
   Pro Ala His Thr Asp Pro Asn Ala Leu Thr Ile Leu Leu Met Asp Pro
       210                 215                 220
   Asp Val Ala Gly Leu Gln Val Leu His Ala Gly Gln Trp Val Ala Val
   225                 230                 235                 240
   Asn Pro Gln Pro Gly Ala Leu Ile Ile Asn Ile Gly Asp Gln Leu Gln
                   245                 250                 255
   Ala Leu Ser Asn Gly Gln Tyr Arg Ser Val Trp His Arg Ala Val Val
               260                 265                 270
   Asn Ser Asp Arg Glu Arg Met Ser Val Ala Ser Phe Leu Cys Pro Cys
           275                 280                 285
   Asn His Val Val Leu Gly Pro Ala Arg Lys Leu Val Thr Glu Asp Thr
   290 295 300
   Pro Ala Val Tyr Arg Asn Tyr Thr Tyr Asp Lys Tyr Tyr Ala Lys Phe
   305 310 315 320
   Trp Ser Arg Asn Leu Asp Gln Glu His Cys Leu Glu Leu Phe Arg Thr
   325 330 335
<210> 84
   <211> 346
   <212> PRT
   <213> Zingiber officinale
<400> 84
   Met Ala Asp Met Leu Leu Ser Ile Gly Glu His Asp Thr Met Pro Arg
   1               5                   10                  15
   Asn Tyr Val Arg Pro Glu Asn Glu Arg Pro His Leu Asp Asn Val Ile
               20                  25                  30
   Ala Asp Ala Asn Ile Pro Val Val Asp Phe Gly Ala Pro Asp Lys Ser
           35                  40                  45
   Gln Ile Ile Ser Gln Ile Glu Lys Ala Cys Arg Leu Tyr Gly Phe Phe
       50                  55                  60
   Gln Val Val Asn His Gly Ile Ala Ala Glu Leu Ile Lys Lys Val Leu
   65                  70                  75                  80
   Ala Ile Ala Leu Glu Phe Phe Arg Leu Pro Gln Glu Glu Lys Ala Lys
                   85                  90                  95
   Leu Tyr Ser Asp Asp Pro Ala Lys Lys Ile Arg Leu Ser Thr Ser Phe
               100                 105                 110
   Asn Val Arg Lys Glu Thr Val His Asn Trp Arg Asp Tyr Leu Arg Leu
           115                 120                 125
   His Cys Tyr Pro Leu Glu Glu Phe Ile Pro Asp Trp Pro Ser Asn Pro
       130                 135                 140
   Ser Ser Phe Lys Asp Val Phe Gly Ser Tyr Cys Gln Gln Val Arg Lys
   145                 150                 155                 160
   Leu Gly Phe Arg Ile Leu Gly Ile Ile Ser Leu Ser Leu Gly Leu Glu
                   165                 170                 175
   Glu Glu Tyr Leu Val Arg Val Leu Gly Glu Gln Glu Gln His Met Ala
               180                 185                 190
   Val Asn Tyr Tyr Pro Lys Cys Pro Glu Pro Glu Leu Thr Tyr Gly Leu
           195                 200                 205
   Pro Ala His Thr Asp Pro Asn Ala Leu Thr Ile Leu Leu Gln Asp Pro
       210                 215                 220
   His Val Ser Gly Leu Gln Val His Lys Asp Gly Lys Trp Ile Ala Val
   225                 230                 235                 240
   Asp Pro Lys Pro Asn Ala Phe Val Ile Asn Ile Gly Asp Gln Leu Gln
                   245                 250                 255
   Ala Leu Ser Asn Gly Arg Tyr Lys Ser Val Trp His Arg Ala Val Val
               260                 265                 270
   Asn Ser Asn Lys Glu Arg Met Ser Val Ala Ser Phe Leu Cys Pro Cys
           275                 280                 285
   Asn Ser Val Leu Ile Ser Pro Pro Glu Lys Leu Ile Ala Asp Gly Cys
       290                 295                 300
   Pro Ala Val Tyr Arg Ser Tyr Thr Tyr Asp Glu Tyr Tyr Lys Lys Phe
   305                 310                 315                 320
   Trp Ser Arg Asn Leu Asp Gln Glu His Cys Leu Glu Leu Phe Lys Lys
                   325                 330                 335
   Glu Arg Glu Thr Cys Pro Asp Ala Pro Thr
               340                 345
<210> 85
   <211> 22
   <212> DNA
   <213> Artificial
<220>
   <223> forward primer AtDMR6_fw
<400> 85
   caccatggcg gcaaagctga ta 22
<210> 86
   <211> 21
   <212> DNA
   <213> Artificial
<220>
   <223> backward primer AtDMR6UTR_rv
<400> 86
   gacaaacaca aaggccaaag a 21
<210> 87
   <211> 24
   <212> DNA
   <213> Artificial
<220>
   <223> forward primer cuc_fw
<400> 87
   caccatgagc agtgtgatgg agat 24
<210> 88
   <211> 20
   <212> DNA
   <213> Artificial
<220>
   <223> backward primer cucUTR_rv
<400> 88
   tgggccaaaa agtttatcca 20
<210> 89
   <211> 27
   <212> DNA
   <213> Artificial
<220>
   <223> forward primer spi_fw
<400> 89
   caccatggca aacaagatat tatccac 27
<210> 90
   <211> 22
   <212> DNA
   <213> Artificial
<220>
   <223> backward primer spiUTR_rv
<400> 90
   ttgctgccta caaaagtaca aa 22
<210> 91
   <211> 24
   <212> DNA
   <213> Artificial
<220>
   <223> forward primer Lsat_fw
<400> 91
   caccatggcc gcaaaagtca tctc 24
<210> 92
   <211> 22
   <212> DNA
   <213> Artificial
<220>
   <223> backward primer LsatUTR_rv
<400> 92
   catggaaaca catattcctt ca 22
<210> 93
   <211> 28
   <212> DNA
   <213> Artificial
<220>
   <223> forward primer Slyc1dmr6_fw
<400> 93
   caccatggaa accaaagtta tttctagc 28
<210> 94
   <211> 20
   <212> DNA
   <213> Artificial
<220>
   <223> backward primer Slyc1dmr6UTR_rv
<400> 94
   gggacatccc tatgaaccaa 20
<210> 95
   <211> 1013
   <212> DNA
   <213> Solanum lycopersicum
<400> 95
   atggaaacca aagttatttc tagcggaatc aaccactcta ctcttcctca aagttacatc      60
   cgacccgaat ccgatagacc acgtctatcg gaagtggtcg attgtgaaaa tgttccaata     120
   attgacttaa gttgcggaga tcaagctcaa ataattcgtc aaattggaga agcttgtcaa     180
   acttatggtt tctttcaggt aattaatcat ggtgtaccaa aggaagttgt agagaaaatg     240
   ctaggggtag ctggggaatt tttcaattta ccagtagaag agaaactaaa attatattca     300
   gatgatcctt caaagaccat gagattatca acaagtttta atgttaaaaa ggagacagtt     360
   cataattgga gagattatct cagacttcat tgttatcctc tagagaagta tgctcctgaa     420
   tggccttcta atccatcatc tttcagggaa atcgtgagca gatattgcag ggaaattcgt     480
   caactcggat ttagattaga agaagccata gcagaaagcc tggggttaga taaagagtgt     540
   ataaaagatg tattgggtga acaaggacaa catatggcta tcaattatta tcctccttgt     600
   ccacaaccag aacttactta tgggcttccg gcccatactg atccaaattc acttacaatt     660
   cttcttcaag acttgcaagt tgcgggtctt caagttctta aagatggcaa atggttagct     720
   gtaaaacctc aacctgacgc ctttgtcatt aatcttgggg atcaattgca ggcagtaagt     780
   aacggtaagt acagaagtgt atggcatcga gctattgtga attcagatca agctaggatg     840
   tcagtggctt cgtttctatg tccgtgtgat agcgcgaaaa tcagtgcacc aaagctgctg     900
   acagaagatg gatctccagt gatttatcaa gactttacgt atgctgagta ttacaacaag     960
   ttctggagca ggaatttgga ccagcaacat tgtttggaac ttttcaagaa taa          1013
<210> 96
   <211> 337
   <212> PRT
   <213> Solanum lycopersicum
<400> 96
   Met Glu Thr Lys Val Ile Ser Ser Gly Ile Asn His Ser Thr Leu Pro
   1               5                   10                  15
   Gln Ser Tyr Ile Arg Pro Glu Ser Asp Arg Pro Arg Leu Ser Glu Val
               20                  25                  30
   Val Asp Cys Glu Asn Val Pro Ile Ile Asp Leu Ser Cys Gly Asp Gln
           35                  40                  45
   Ala Gln Ile Ile Arg Gln Ile Gly Glu Ala Cys Gln Thr Tyr Gly Phe
       50                  55                  60
   Phe Gln Val Ile Asn His Gly Val Pro Lys Glu Val Val Glu Lys Met
   65                  70                  75                  80
   Leu Gly Val Ala Gly Glu Phe Phe Asn Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ser Ser Phe Arg Glu Ile Val Ser Arg Tyr Cys Arg Glu Ile Arg
   145                 150                 155                 160
   Gln Leu Gly Phe Arg Leu Glu Glu Ala Ile Ala Glu Ser Leu Gly Leu
                   165                 170                 175
   Asp Lys Glu Cys Ile Lys Asp Val Leu Gly Glu Gln Gly Gln His Met
               180                 185                 190
   Ala Ile Asn Tyr Tyr Pro Pro Cys Pro Gln Pro Glu Leu Thr Tyr Gly
               195                 200                 205
   Leu Pro Ala His Thr Asp Pro Asn Ser Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Lys Tyr Arg Ser Val Trp His Arg Ala Ile
               260                 265                 270
   Val Asn Ser Asp Gln Ala Arg Met Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Asn Lys
   305                 310                 315                 320
   Phe Trp Ser Arg Asn Leu Asp Gln Gln His Cys Leu Glu Leu Phe Lys
                   325                 330                 335
   Asn
<210> 97
   <211> 1014
   <212> DNA
   <213> Nicotiana benthamiana
<400> 97
   atggaagcaa aagttctttc cagcggaatc cgccactcta ctatccctca aagttacatc     60
   cgccctcaat ccgataggcc gcgcctttct gaagttgctg attgtgaaaa cgttccagta    120
   gttgatatag gttgcggtga tagaaacctt attgttcatc aaattggtga agcctgtcgt    180
   ctttatggtt ttttccaggt aattaatcat ggtgtaccaa agaatttaat agacgaaatg    240
   ctagagatag ctggggaatt ttttaggctt ccagttgaag agaagttgaa attgtactca    300
   gatgacccat cgaagacgat gagattgtcg actagtttta atgtgaaaaa ggagaaggtt    360
   cacaattgga gagattatct cagacttcat tgttatcctc ttgaaaatta cgctcctgaa    420
   tggccttcca atccttcctc tttcagggaa atcgtgagca gatattgcat ggaagttcga    480
   caactcgggt tcagattgca ggaagccata gcagagagcc taggcttaga gaaagagtgt    540
   ataaaggatg tattgggcga acaaggtcaa cacatggcta tcaatttcta tcctccttgt    600
   ccacaaccag aactcactta tgggctgcca gcacatactg atccaaatgc ccttacaatt    660
   cttcttcaag acttagaagt agctggtctt caagttctta aagatggcga atggttggcc    720
   gtcaagcctc aaccagatgc ctttgtcatt aatcttggtg atcaactgca ggcagtgagt    780
   aatgggagat acaaaagcgt atggcatcga gctattgtaa attcagacaa agccaggttg    840
   tcagtggctt cgttcctttg tccgtgcgat agcgcgaaaa tcagtgctcc aaagctcctc    900
   actgaagatg gatctcctgt catttatcag gactttacct atgctgagta ttacaaaaag    960
   ttctggagca ggaatttgga ccaggaacat tgtttggaac ttttcaagaa ctaa         1014
<210> 98
   <211> 337
   <212> PRT
   <213> Nicotiana benthamiana
<400> 98
   Met Glu Ala Lys Val Leu Ser Ser Gly Ile Arg His Ser Thr Ile Pro
   1               5                   10                  15
   Gln Ser Tyr Ile Arg Pro Gln Ser Asp Arg Pro Arg Leu Ser Glu Val
               20                  25                  30
   Ala Asp Cys Glu Asn Val Pro Val Val Asp Ile Gly Cys Gly Asp Arg
           35                  40                  45
   Asn Leu Ile Val His Gln Ile Gly Glu Ala Cys Arg Leu Tyr Gly Phe
       50                  55                  60
   Phe Gln Val Ile Asn His Gly Val Pro Lys Asn Leu Ile Asp Glu Met
   65                  70                  75                  80
   Leu Glu Ile Ala Gly Glu Phe Phe Arg Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Lys Val His Asn Trp Arg Asp Tyr Leu Arg
           115                 120                 125
   Leu His Cys Tyr Pro Leu Glu Asn Tyr Ala Pro Glu Trp Pro Ser Asn
       130                 135                 140
   Pro Ser Ser Phe Arg Glu Ile Val Ser Arg Tyr Cys Met Glu Val Arg
   145                 150                 155                 160
   Gln Leu Gly Phe Arg Leu Gln Glu Ala Ile Ala Glu Ser Leu Gly Leu
                   165                 170                 175
   Glu Lys Glu Cys Ile Lys Asp Val Leu Gly Glu Gln Gly Gln His Met
               180                 185                 190
   Ala Ile Asn Phe Tyr Pro Pro Cys Pro Gln Pro Glu Leu Thr Tyr Gly
           195                 200                 205
   Leu Pro Ala His Thr Asp Pro Asn Ala Leu Thr Ile Leu Leu Gln Asp
       210                 215                 220
   Leu Glu Val Ala Gly Leu Gln Val Leu Lys Asp Gly Glu Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Arg Tyr Lys Ser Val Trp His Arg Ala Ile
               260                 265                 270
   Val Asn Ser Asp Lys Ala Arg Leu Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Lys Lys
   305                 310                 315                 320
   Phe Trp Ser Arg Asn Leu Asp Gln Glu His Cys Leu Glu Leu Phe Lys
                   325                 330                 335
   Asn

## Description

The present invention relates to disease resistant melon plants, in particular melon plants resistant to *Pseudoperonospora cubensis.*

Resistance of plants to fungal and oomycete pathogens has been extensively studied, for both pathogen specific and broad resistance. In many cases resistance is specified by dominant genes for resistance. Many of these race-specific or gene-for-gene resistance genes have been identified that mediate pathogen recognition by directly or indirectly interacting with avirulence gene products or other molecules from the pathogen. This recognition leads to the activation of a wide range of plant defence responses that arrest pathogen growth.

In plant breeding there is a constant struggle to identify new sources of mostly monogenic dominant resistance genes. In cultivars with newly introduced single resistance genes, protection from disease is often rapidly broken, because pathogens evolve and adapt at a high frequency and regain the ability to successfully infect the host plant. Therefore, the availability of new sources of disease resistance is highly needed.

Alternative resistance mechanisms act for example through the modulation of the defence response in plants, such as the resistance mediated by the recessive *mlo* gene in barley to the powdery mildew pathogen *Blumeria graminis f.sp. hordei.* Plants carrying mutated alleles of the wildtype *MLO* gene exhibit almost complete resistance coinciding with the abortion of attempted fungal penetration of the cell wall of single attacked epidermal cells. The wild type *MLO* gene thus acts as a negative regulator of the pathogen response. This is described in WO9804586.

Other examples are the recessive powdery mildew resistance genes, found in a screen for loss of susceptibility to *Erysiphe cichoracearum.* Three genes have been cloned so far, named *PMR6,* which encodes a pectate lyase-like protein, *PMR4* which encodes a callose synthase, and *PMR5* which encodes a protein of unknown function. Both *mlo* and *pmr* genes appear to specifically confer resistance to powdery mildew and not to oomycetes such as downy mildews.

Broad pathogen resistance, or systemic forms of resistance such as SAR, has been obtained by two main ways. The first is by mutation of negative regulators of plant defence and cell death, such as in the *cpr, lsd* and *acd* mutants of *Arabidopsis.* The second is by transgenic overexpression of inducers or regulators of plant defence, such as in *NPR1* overexpressing plants.

The disadvantage of these known resistance mechanisms is that, besides pathogen resistance, these plants often show detectable additional and undesirable phenotypes, such as stunted growth or the spontaneous formation of cell death.

It is an object of the present invention to provide a form of resistance that is broad, durable and not associated with undesirable phenotypes.

In the research that led to the present invention, an *Arabidopsis thaliana* mutant screen was performed for reduced susceptibility to the downy mildew pathogen *Hyaloperonospora parasitica.* EMS-mutants were generated in the highly susceptible *Arabidopsis* line L*er eds1-2.* Eight downy mildew resistant (*dmr*) mutants were analysed in detail, corresponding to 6 different loci. Microscopic analysis showed that in all mutants *H. parasitica* growth was severely reduced. Resistance of *dmr3, dmr4* and *dmr5* was associated with constitutive activation of plant defence. Furthermore, the *dmr3* and *dmr4,* but not *dmr5* mutants, were also resistant to *Pseudomonas syringae* and *Golovinomyces orontii.*

In contrast, enhanced activation of plant defence was not observed in the *dmr1, dmr2,* and *dmr6* mutants. The results of this research have been described in Van Damme et al. (2005) Molecular Plant-Microbe Interactions 18(6) 583-592. This article does not disclose the identification and characterization of the DMR genes.

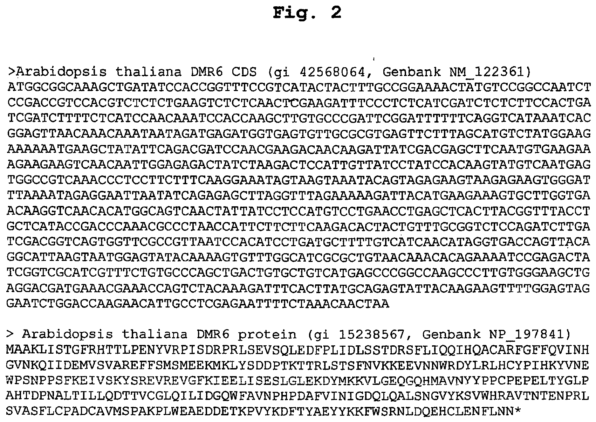

The *dmr6* mutant was identified in a loss-of-susceptibility screen in the Arabidopsis *Ler eds1-2* background. The *DMR6* gene now has been cloned and characterized. Thus, it was found that *DMR6* is the gene At5g24530, encoding for an oxidoreductase (DNA and amino acid sequence are depicted in **Figure 2**). Oxidoreductases are enzymes that catalyze the transfer of electrons from one molecule, the oxidant, to another, the reductant. According to the present invention, it has been found that lack of a functional DMR6 protein results in downy mildew resistance.

The present invention provides a melon plant, which is resistant *Pseudoperonospora cubensis* as defined in the appended claims.

The resistance according to the invention is based on an altered, in particular a reduced level or complete absence of the DMR6 protein *in planta.* The term "DMR6 protein" in this respect relates to the *DMR6* gene product. Such alterations can be achieved in various ways.

In one embodiment of the invention, the reduced level of DMR6 protein is the result of a reduced endogenous *DMR6* gene expression. Reducing the expression of the DMR6 gene can be achieved, either directly, such as by gene silencing, or indirectly by modifying the regulatory sequences thereof, or by stimulating repression of the gene.

Modulating the DMR6 gene to lower its activity or expression can be achieved at various levels. First, the endogenous gene can be directly mutated. This can be achieved by means of a mutagenic treatment. Alternatively, a modified DMR6 gene can be brought into the plant by means of transgenic techniques or by introgression, or the expression of DMR6 can be reduced at the regulatory level, for example by modifying the regulatory sequences or by gene silencing.

In another embodiment of the invention, the reduced level of DMR6 protein is the result of a mutation in the *DMR6* gene resulting in a reduced DMR6 expression as compared to the wild-type DMR6 gene wherein no such mutation is present, or resulting in a reduced mRNA or protein stability. In a particular embodiment this is achieved by mutations in the DMR6 coding sequence that result in a non-functional DMR6 protein. In another embodiment of the invention, reduced expression can be achieved by down-regulation of *DMR6* gene expression either at the transcriptional or the translational level, e.g. by gene silencing or by mutations that affect the expression of the *DMR6* gene.

This invention is based on research performed on resistance to *Hyaloperonospora parasitica* in *Arabidopsis* but is a general concept that can be more generally applied in plants, in particular in crop plants that are susceptible to infections with pathogens, such as Oomycota and Fungi.

The invention is suitable for a large number of plant diseases caused by oomycetes such as*Pseudoperonospora cubensis* on melon.

When the modification of *DMR6* gene expression in a plant is to be achieved via genetic modification of the DMR6 gene or via the identification of mutations in the DMR6 gene, and the gene is not yet known it must first be identified. To generate pathogen-resistant plants, in particular crop plants, via genetic modification of the DMR6 gene or via the identification of mutations in the DMR6 gene, the orthologous DMR6 genes must be isolated from these plant species.

Various methods are available for the identification of orthologous sequences in other plants.

A method for the identification of DMR6 orthologous sequences in a plant species, may for example comprise identification of DMR6 ESTs of the plant species in a database; designing primers for amplification of the complete DMR6 transcript or cDNA; performing amplification experiments with the primers to obtain the corresponding complete transcript or cDNA; and determining the nucleotide sequence of the transcript or cDNA. Suitable methods for amplifying the complete transcript or cDNA in situations where only part of the coding sequence is known are the advanced PCR techniques 5'RACE, 3'RACE, TAIL-PCR, RLM-RACE and vectorette PCR.

Alternatively, if no nucleotide sequences are available for the plant species of interest, primers are designed on the DMR6 gene of a plant species closely related to the plant of interest, based on conserved domains as determined by multiple nucleotide sequence alignment, and used to PCR amplify the orthologous sequence. Such primers are suitably degenerate primers.

Another reliable method to assess a given sequence as being a DMR6 ortholog is by identification of the reciprocal best hit. A candidate orthologous DMR6 sequence of a given plant species is identified as the best hit from DNA databases when searching with the *Arabidopsis* DMR6 protein or DNA sequence, or that of another plant species, using a Blast programme. The obtained candidate orthologous nucleotide sequence of the given plant species is used to search for homology to all *Arabidopsis* proteins present in the DNA databases (e.g. at NCBI or TAIR) using the BlastX search method. If the best hit and score is to the *Arabidopsis* DMR6 protein, the given DNA sequence can be described as being an ortholog, or orthologous sequence.

DMR6 is encoded by a single gene in *Arabidopsis* as deduced from the complete genome sequence that is publicly available. In the genome of rice 3 orthologs, and in poplar 2 orthologs have been identified. In most other plant species tested so far, DMR6 appears to be encoded by a single gene, as determined by the analysis of mRNA sequences and EST data from public DNA databases. The orthologous genes and proteins are identified in these plants by nucleotide and amino acid comparisons with the information that is present in public databases.

Alternatively, if no DNA sequences are available for the desired plant species, orthologous sequences are isolated by heterologous hybridization using DNA probes of the DMR6 gene of *Arabidopsis* or another plant or by PCR methods, making use of conserved domains in the DMR6 coding sequence to define the primers. For many crop species, partial DMR6 mRNA sequences are available that can be used to design primers to subsequently PCR amplify the complete mRNA or genomic sequences for DNA sequence analysis.

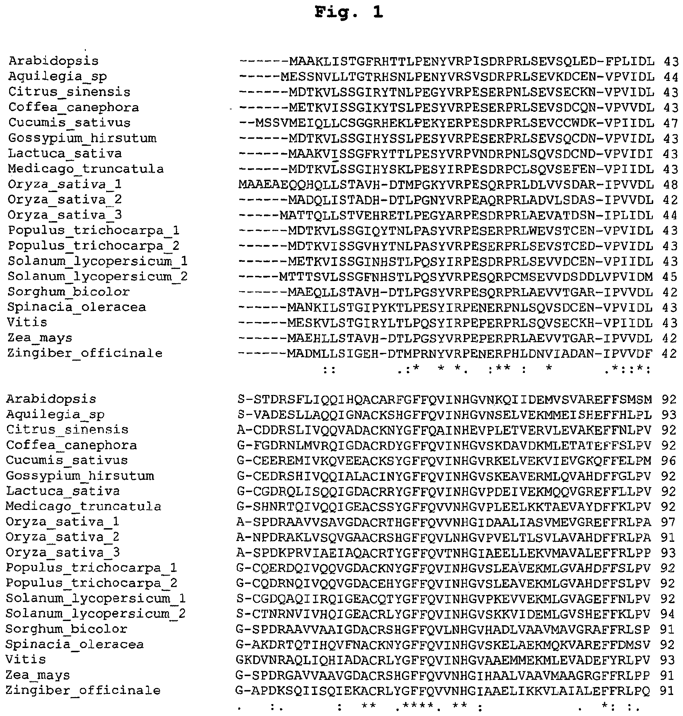

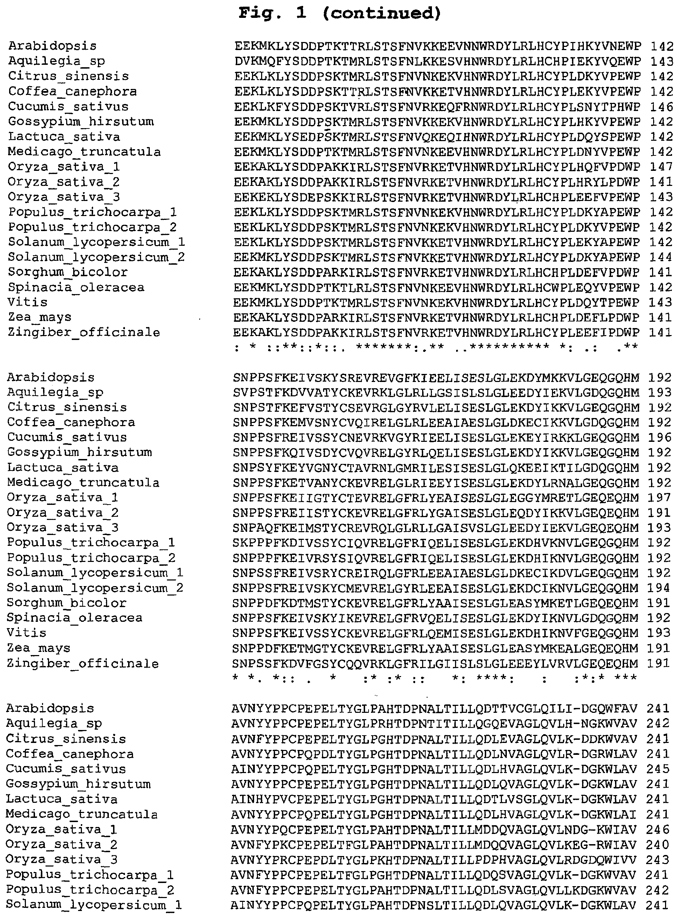

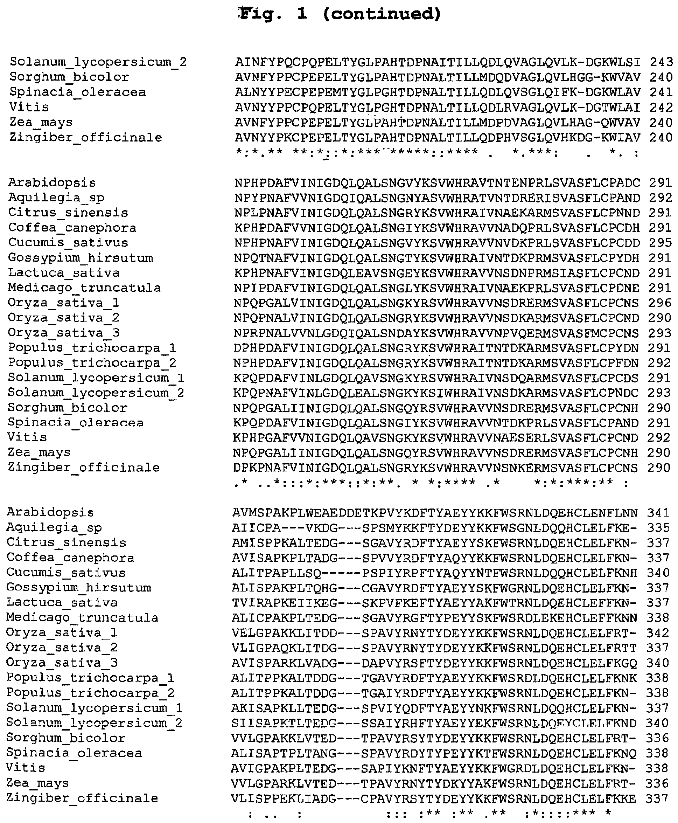

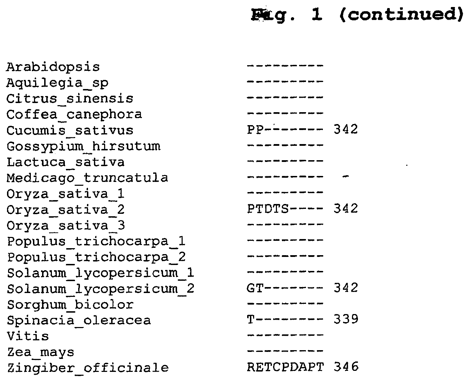

**Figure 1** shows orthologous DMR6 sequences (described in Table 1) that have been identified in publicly available databases and obtained by PCR amplification on cDNA and subsequent sequencing. After orthologous DMR6 sequences are identified, the complete nucleotide sequence of the regulatory and coding sequence of the gene is identified by standard molecular biological techniques. For this, genomic libraries of the plant species are screened by DNA hybridization or PCR with probes or primers derived from a known *DMR6* gene to identify the genomic clones containing the *DMR6* gene. Alternatively, advanced PCR methods, such as RNA ligase-mediated RACE (RLM-RACE), can be used to directly amplify gene and cDNA sequences from genomic DNA or reverse-transcribed mRNA. DNA sequencing subsequently results in the characterization of the complete gene or coding sequence.

Once the DNA sequence of the gene is known this information is used to prepare the means to modulate the expression of the *DMR6* gene.

To achieve a reduced DMR6 protein level, the expression of the DMR6 gene can be down-regulated or the enzymatic activity of the DMR6 protein can be reduced by amino acid substitutions resulting from nucleotide changes in the DMR6 coding sequence.

Downregulation of DMR6 gene expression can be achieved by gene-silencing using RNAi. For this, transgenic plants are generated expressing a DMR6 anti-sense construct, an optimized micro-RNA construct, an inverted repeat construct, or a combined sense-anti-sense construct, so as to generate dsRNA corresponding to DMR6 that leads to gene silencing.

One or more regulators of the DMR6 gene can be downregulated (in case of transcriptional activators) by RNAi.

Regulators can be upregulated (in case of repressor proteins) by transgenic overexpression. Overexpression is achieved in a particular embodiment by expressing repressor proteins of the DMR6 gene from a strong promoter, e.g. the 35S promoter that is commonly used in plant biotechnology.

The downregulation of the DMR6 gene can also be achieved by mutagenesis of the regulatory elements in the promoter, terminator region, or potential introns. Mutations in the *DMR6* coding sequence in many cases leads to amino acid substitutions or premature stop codons that negatively affect the expression or activity of the encoded DMR6 protein.

These mutations are induced in plants by using mutagenic chemicals such as ethyl methane sulfonate (EMS), by irradiation of plant material with gamma rays or fast neutrons, or by other means. The resulting nucleotide changes are random, but in a large collection of mutagenized plants the mutations in the DMR6 gene can be readily identified by using the TILLING (Targeting Induced Local Lesions IN Genomes) method (McCallum et al. (2000) Targeted screening for induced mutations. Nat. Biotechnol. 18,455-457, and Henikoff et al. (2004) TILLING. Traditional mutagenesis meets functional genomics. Plant Physiol. 135, 630-636). The principle of this method is based on the PCR amplification of the gene of interest from genomic DNA of a large collection of mutagenized plants in the M2 generation. By DNA sequencing or by looking for point mutations using a single-strand specific nuclease, such as the CEL-I nuclease (Till et al. (2004) Mismatch cleavage by single-strand specific nucleases. Nucleic Acids Res. 32, 2632-2641) the individual plants that have a mutation in the gene of interest are identified.

By screening many plants, a large collection of mutant alleles is obtained, each giving a different effect on gene expression or enzyme acitivity. The gene expression or protein levels can for example be tested by analysis of DMR6 transcript levels (e.g. by RT-PCR) or by quantification of DMR6 protein levels with antibodies.

Plants with the desired reduced DMR6 level or *DMR6* expression are then back-crossed or crossed to other breeding lines to transfer only the desired new allele into the background of the crop wanted.

The present invention demonstrates that plants having no or a reduced level of functional DMR6 gene product show resistance to pathogens, in particular of oomycete and fungal origin. With such knowledge the skilled person can identify so far unknown natural variants of a given plant species that have variants of the DMR6 gene that lead to a reduced level or absence of a functional DMR6 protein, or mutated versions of the DMR6 protein.

Disclosed is the use of a DMR6 promotor for providing disease resistance into plants, i.e. for providing plants with a resistance to a pathogen of viral, bacterial, fungal or oomycete origin. The transcriptional up-regulation of DMR6 in response to pathogen infection has been demonstrated. Both transcript analysis as well as promotor DMR6-reporter lines support this finding (see Example 1, below). The pathogen-inducible DMR6 promotor according to the invention thus is particularly useful to control the expression of inducible systems that lead to disease resistance in plants.

One example of such inducible system that leads to disease resistance in plants, and in which the DMR6 promotor may be effective, has e.g. been described in WO 99/45125, wherein an antisense nucleotide sequence for a gene involved in the regulation of the C-5 porphyrin metabolic pathway is operably linked to a pathogen-inducible promotor and used to transform plant cells. Expression of the antisense nucleotide sequence in response to the pathogen effectively disrupts porphyrin metabolism of the transformed plant cell, and development of a localized lesion wherein the spread of the pathogen is contained. WO 96/36697 also discloses inducible systems leading to disease resistance in plants, wherein an inducible promotor controls the expression of a protein capable of evoking the hypersensitivity response in a plant. EP 0474857 furthermore discloses a method for the induction of pathogen resistance in plants, comprising transforming plants with polynucleotide sequences encoding a pair of pathogen-derived-avirulence-gene/plant-derived-resistance gene, wherein the expression of one of or both the elicitor peptide and the resistance gene is regulated by a pathogen inducible promotor. Further examples of inducible systems leading to resistance to pathogens in plants have been described in e.g. WO 98/32325.

Disclosed is a method of providing disease resistance in a plant, comprising transforming a plant cell with a DNA construct comprising at least one expressible nucleic acid which is operably linked to a pathogen-inducible promotor that is operable within a plant cell, and regenerating transformed plants from said plant cells, wherein the pathogen-inducible promotor is a DMR6 promotor, and wherein the expression of the expressible nucleic acid confers disease resistance to the transgenic plant.

Disclosed are disease resistance plants, obtainable by said method, as well as to plant tissue, and seeds obtained from said plants.

Disclosed are plants, which are resistant to a pathogen of viral, bacterial, fungal or oomycete origin, wherein the plant comprises in its genome a DNA construct, comprising at least one expressible nucleic acid which is operably linked to a pathogen-inducible promotor, wherein the pathogen-inducible promotor is a DMR6 promotor.

Disclosed is the DNA construct per se, comprising at least one expressible nucleic acid which is operably linked to a pathogen-inducible promotor, wherein the pathogen-inducible promotor is a DMR6 promotor. The construct of the invention can be used to transform plant cells which may be regenerated into transformed plants. Furthermore, transformed plant tissue and seed may be obtained. Suitable methods for introducing the construct into plant cells are known to the skilled person.

According to the invention, by "operably linked" is meant that a promotor and an expressible nucleic acid, e.g. a gene, are connected in such way as to permit initiation of transcription of the expressible nucleic acid (e.g. gene) by the promotor.

By "expressible nucleic acid" is meant a nucleic acid (e.g. a gene, or part of a gene) that can be expressed in the cell, i.e. that can be transcribed into mRNA, and eventually may be translated into a protein. The expressible nucleic acid may be genomic DNA, cDNA, or chemically synthesized DNA or any combination thereof.

A DNA construct comprises all necessary nucleic acid elements which permit expression (i.e. transcription) of a particular nucleic acid in a cell. Typically, the construct includes an expressible nucleic acid, i.e. a nucleic acid to be transcribed, and a promotor. The construct can suitably be incorporated into e.g a plasmid or vector.

The expressible nucleic acid preferably is a gene involved in a plant defence response, e.g. a gene associated with the hypersensitivity response of a plant. In the hypersensitivity response (HR) of a plant, the site in the plant where the pathogen invades undergoes localized cell death by the induced expression of a suicide mechanism that triggers said localized cell death in response to pathogens. In this way, only a few plant cells are sacrificed and the spread of the pathogen is effectively arrested. Examples of said genes involved in a plant defence response are the regulatory protein NPR1/NIM1 (Friedrich et al., Mol. Plant Microbe Interact. 14(9): 1114-1124,2001) and the transcription factor MYB30 (Vailleau et al., Proc. Natl. Acad. Sci. USA 99(15): 10179-10184, 2002).

The expressible nucleic acid can encode an autologous or heterologous polypeptide capable of conferring disease-resistance to a plant. By "autologous polypeptide" is meant any polypeptide that is expressed in a transformed plant cell from a gene that naturally occurs in the transformed plant cell. By "heterologous polypeptide" is meant any polypeptide that is expressed in a transformed plant cell from a gene that is partly or entirely foreign (i.e. does not naturally occur in) to the transformed plant cell. Examples of such polypeptides are the mammalian Bax protein, which encodes a pro-apoptotic protein and results in cell death in plants (Lacomme and Santa Cruz, Proc. Natl. Acad. Sci. USA 96(14): 7956-61, 1999) and fungal chitinases (de las Mercedes Dana et al., Plant Physiol. 142(2): 722-730, 2006).

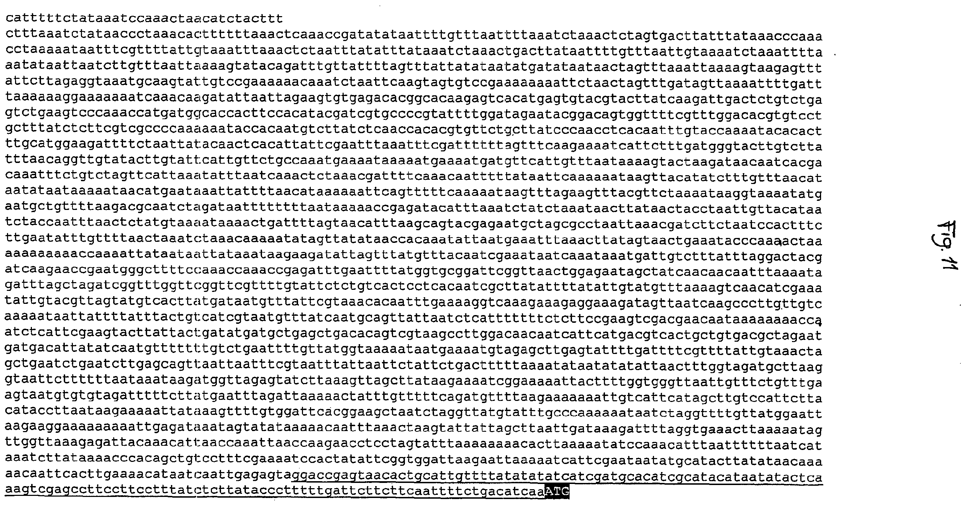

The DMR6 promotor can be the Arabidopsis DMR6 promotor. The DMR6 promotor comprises a region of 3000 bp that is upstream of the Arabidopsis *DMR6* coding sequence (ATG start codon) and includes the 5'UTR. Preferably the DMR6 promotor comprises a nucleotide sequence as defined in **Figure 11****,** and/or any functional fragment thereof, i.e. any fragment (or part) of said sequence which still is capable of initiating transcription of the expressible nucleic acid(s) to which it is operably linked, and/or natural variants thereof, i.e. natural variants of this promotor which may contain small polymorphisms, but which are generally at least 90% identical.

The DMR6 promotor can be an orthologous DMR6 promotor, i.e. a promotor of an orthologous DMR6 gene. Methods for identifying DMR6 orthologs have been described in Example 2 below. Once the DMR6 orthologs have been identified, the skilled person will be able to isolate the respective promotor of said orthologs, using standard molecular biological techniques.

The DMR6 promotor has been shown to be strongly pathogen-induced, and the DMR6 promotor is not highly expressed in other non-infected tissues. Thus, it is a very suitable promotor for use in inducible systems for providing resistance to pathogens of viral, bacterial, fungal or oomycete origin in plants. Examples of specific pathogens and plants for which the inducible system, using the DMR6 promotor suitably can be used, have been given above.

The present invention is illustrated in the following examples that are not intended to limit the invention in any way. In the examples reference is made to the following figures.
**Table 1** shows the Genbank accession numbers and Genlnfo identifiers of the *Arabidopsis* DMR6 mRNA and orthologous sequences from other plant species.
**Table 2** shows the PCR primers for the markers used for the map-based cloning of DMR6.
**Table 3** shows primer pairs for cloning *dmr6* orthologs in a suitable plant expression vector.
**Figure 1** shows the alignment of the amino acid sequences of the DMR6 protein of *Arabidopsis thaliana* and orthologs from *Aquilegia species, Citrus sinensis, Coffea canephora, Cucumis sativus, Gossypium hirsitum, Lactuca sativa, Medicago truncatula, Oryza sativa (3), Populus trichocarpa (2), Solanum lycopersicum (2), Sorghum bicolor, Spinacia oleracea, Vitis vinifera, Zea mays,* and *Zingiber officinale,* using the CLUSTAL W (1.83) multiple sequence alignment programme (EBI). Below the sequences the conserved amino acids are indicated by the dots, and the identical amino acids are indicated by the asteriks.
**Figure 2** shows the nucleotide and amino acid sequence of the DMR6 gene (At5g24530, gi 42568064, Genbank NM_122361) and protein (gi 15238567, Genbank NP_197841) of *Arabidopsis thaliana,* respectively.
**Figure 3** shows the nucleotide and derived amino acid sequence of the DMR6 ortholog of *Lactuca sativa,* respectively.
**Figure 4** shows the nucleotide and derived amino acid sequence of the DMR6 ortholog of *Spinacia oleracea,* respectively.
**Figure 5** shows the nucleotide and derived amino acid sequence of the DMR6 ortholog of *Cucumis sativus* and *Cucumis melo.*
**Figure 6** shows the downy mildew resistance of the *Arabidopsis dmr6* mutants. (a) Quantification of sporangiophores of *H. parasitica* isolate Waco9, 7 days post inoculation, on the *dmr6-1* mutant (BC₂, line E37) compared to its parental line L*er eds1-2* and on the *dmr6-2* mutant (FLAG_445009 T-DNA line) compared to its parental line Ws-4. (b) Restoration of susceptibility by complementation with the At5g24530 gene in the *dmr6-1* mutant. *H. parasitica* spores per mg seedling weight were quantified on *Ler eds1-2, dmr6-1* and 5 complementation lines (#121, 122, 211,231, and 241).
**Figure 7** shows the structure of the *Arabidopsis DMR6* gene and *dmr6-1* and *dmr6-*2 mutations. The *DMR6* gene contains four exons and a coding sequence of 1026 bases. The two alleles are indicated; *dmr6-1* with a base change in exon 2, and *dmr6-2* with a T-DNA insertion into intron 2.
**Figure 8** shows the relative transcript levels of *DMR6* in L*er* plants either mock treated or inoculated with a compatible or incompatible *H. parasitica* isolate. Transcript levels were determined at different days post inoculation. The difference in cycle threshold (ΔCT) values reflect the number of additional PCR amplification cycles required to reach an arbitrary threshold product concentration as compared to *ACTIN2.* A lower ΔCT value indicates a higher transcript level.
**Figure 9** shows the expression of the DMR6 promoter-reporter (pDMR6::GUS) construct in transgenic Arabidopsis lines, visualized with only X-gluc as substrate (Figure d and e) or Magenta-Xgluc (Figure a-c) and trypan blue staining of *H. parasitica* growth (a) L*er eds1-2* (p*DMR6*::GUS) 3dpi with *H. parasitica,* Cala2 isolate. (b) Col-0 (p*DMR6*::GUS) 3dpi with *H. parasitica,* Waco9 isolate. (c) L*er eds1-2* (p*DMR6*::GUS) 3dpi with *H. parasitica,* Emoy2 isolate. (d) Col-0 (p*DMR6*::GUS) 3 dp wounding. (e) Col-0 (p*DMR6*::GUS) 3 dp BTH application.
**Figure 10** shows the Q-PCR analysis of the transcript levels of the genes; At4g14365, At1g14880, *ACD6, PR-1, PR-2* and *PR-5,* selected as up regulated in the *dmr6-1* micro array analysis.(a) Transcription levels of the six genes in *dmr6-1* compared to L*er eds1-2* and additionally the *DMR6* transcript. (b) Elevated gene transcripts of six defence-associated genes in *dmr6-2* versus Ws-4. ΔCT reflects the number of additional PCR amplification cycles required to reach the level of *ACTIN2* transcripts. A lower ΔCT value indicates a higher transcript level.
**Figure 11** shows the nucleotide sequence of the 3 kb region upstream of the start codon of the DMR6 gene, (at5g24530) of Arabidopsis thaliana, including the promotor and 5'-UTR (underlined).
**Figure 12** shows the nucleotide and derived amino acid sequence of the DMR6 ortholog of *Solanum lycopersicum,* respectively.
**Figure 13** shows the nucleotide and derived amino acid sequence of the DMR6 ortholog of *Nicotiana benthamiana,* respectively.
**Figure 14** shows complementation of Arabidopsis thaliana *dmr6-1* with DMR6 derived from *Cucumis sativa* (Cs), *Spinacia oleracea* (Si), *Lactuca sativa* (Ls) and *Solanum lycopersicum* (So).

### EXAMPLE 1

### The Arabidopsis DMR6 (At5g24530) gene is required for downy mildew susceptibility

### Experimental procedures

### Hyaloperonospora parasitica growth and infection

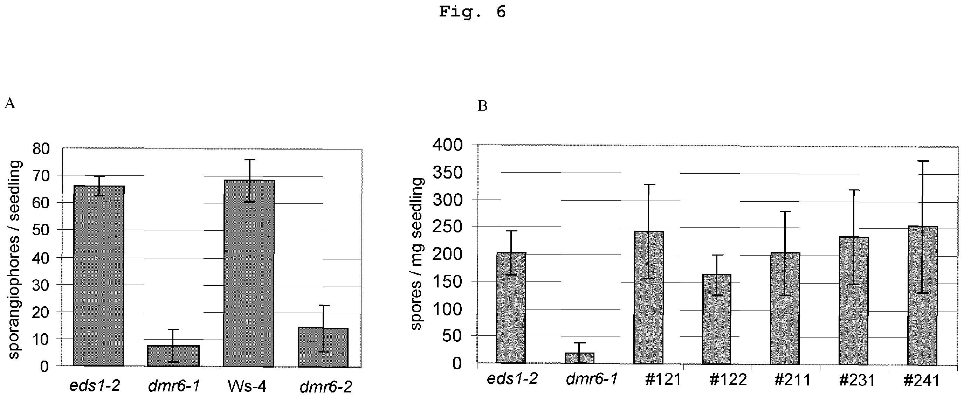

*H. parasitica* isolate Waco9 was provided by Dr. M. Aarts (WUR, Wageningen, NL) and isolate Cala2 provided by Dr. E. Holub (Warwick HRI, Wellsbourne, UK) and maintained on *Arabidopsis* Ws-0 and L*er*, respectively. Inocula (400.000 spores per ml) were weekly transferred to 10 day old healthy seedlings (Holub, E. B. et al., Mol. Plant Microbe Interact. 7: 223-239, 1994) by use of a spray gun. Seedlings were air-dried for approximately 45 minutes and incubated under a sealed lid at 100% relative humidity in a growth chamber at 16°C with 9 hours of light per day (100mE/m2/s). The sporulation levels were quantified 7 days post inoculation (dpi) by counting the number of sporangiophores per seedling, for at least 40 seedlings per tested line (**Figure 6a**) or by isolating spores in water 5 dpi and determining the spore concentration to give the number per mg leaf tissue (**Figure 6b**).

### Generation of backcrossed dmr6 lines

The *dmr6* mutants were back crossed twice (BC₂) to the parental line L*er eds1-2* as well as L*er*. The BC₂ lines generated with Lerwere selected for the presence of the wild type *EDS1* gene by PCR analysis.

### Cloning DMR6

Fine mapping of the *dmr6* gene was done with PCR markers designed using the Cereon database to identify insertion and deletion (IND) differences between Col-0 and L*er*. The markers: IND_MOP9 in gene At5G24210; IND_K16H17 in gene At5G24420; IND_T4C12 in gene At5G24820; IND_T11H3 in between genes At5G24950_60 and IND_F21J6 in gene At5G25270 were used for mapping (Table 2). An additional screen for new recombinants was initiated on 300 F₂ plants resulting in eight F₂ recombinant plants between the two IND based markers IND_MOP9 and IND_T4C12, which flanked a region of 61 genes. Seven additional markers (M450-M590; Table 2) reduced the region to eighteen candidate genes for the *dmr6* locus, between At5g24420 and At5g24590. Sequence analysis of At5g24530 indicated a point mutation leading to a stop codon in exon 2 in the *dmr6-1* mutant.

### Identification of a dmr6 T-DNA insertion line

A second *dmr6* allele was identified, 445D09 a FLAG T-DNA insertion line generated by INRA Versailles in the Ws-4 accession background. The T-DNA insertion was confirmed by PCR using a primer designed in the At5g24530 gene, LP primer (5'-caggtttatggcatatctcacgtc-3'), in combination with the T-DNA right border primer, Tag3' (5'-tgataccagacgttgcccgcataa-3') or RB4 (5'-tcacgggttggggtttctacaggac-3'). The exact T-DNA insertion in the second intron of At5g24530 was confirmed by sequencing of amplicons generated with the T-DNA primers from both the left and right border in combination with the gene specific primers LP or RP (5'-atgtccaagtccaatagccacaag-3').

### cDNA synthesis

RNA was isolated (from approximately 100 mg leaf tissue from 10 day old seedlings) with the RNaesy kit (Qiagen, Venlo, The Netherlands) and treated with the RNase-free DNase set (Qiagen). Total RNA was quantified using an UVmini-1240 spectrophotometer (Shimadzu, Kyoto, Japan). cDNA was synthesized with Superscript III reverse transcriptase (Invitrogen, Carlsbad, CA, USA) and oligo(dT)15 (Promega, Madison, WI, USA), according manufactures instructions.

### Complementation of the dmr6-1 mutant

Complementation lines were generated by transforming *dmr6* plants by the floral dip method with *Agrobacterium tumefaciens* (Clough and Bent, 1998) containing the At5g24530 gene from Col-0 behind the 35S promoter. The construct was generated by PCR amplification of the full length At5g24530 from Col-0 cDNA with primers which included restriction sites that were used for directional cloning. A forward primer (5'-ttctgggtccaATGGCGGCAAAGCTGATATC-3') containing a BamHl restriction site near the start codon (ATG), amplified the 5'-end of *DMR6* and at the 3'-end after the stop codon an EcoRI site was generated with a reverse primer (5'-gatatatgaattcttagttgtttagaaaattctcgaggc-3'). The 35S-*DMR6*-Tn was cloned into the pGreenII0229 (Hellens,R.P., Edwards,E.A., Leyland,N.R., Bean,S., and Mullineaux,P.M. (2000)). pGreen: a versatile and flexible binary Ti vector for Agrobacterium-mediated plant transformation. Plant Mol. Biol. 42, 819-832). 300 µM DL-Phosphinothricin (BASTA) resistant seedlings were isolated and analyzed for *H. parasitica* susceptibility and for DMR6 expression levels by RT-PCR.

### Knock down lines of DMR6 by RNAi

RNAi lines were generated in the L*er eds1-2* and Col-0 background. A 782 bp long cDNA amplicon of Col-0 At5g24530 gene was generated. The PCR was done with the Phusion DNA polymerase (2U/µL) and two different primer combinations. The amplicon from the first DMR6 gene specific primer combination (RNAiDMR6F: 5'- aaaagcaggctGACCGTCCACGTCTCTCTGAA -3' and RNAiDMR6R: 5'- AGAAAGCTGGGTGAAACGATGCGACCGATAGTC-3') was used as a template for the second PCR amplificaton with general primers allowing recombination into the pDONR7 vector of the GateWay cloning system. For the second PCR 10 µl of the first PCR (denaturation for 30 sec. at 98 °C followed by 10 cycles of: 10 sec. at 98°C; 30 sec. at 58 °C; 30 sec. at 72°C) in a total volume of 20 µl was used as template. The second PCR (denaturation for 30 sec. at 98 °C followed by 5 cycles of: 10 sec. at 98°C; 30 sec. at 45 °C; 30 sec. at 72°C and 20 cycles of 10 sec. at 98°C; 30 sec. at 55 °C; 30 sec. at 72°C finished by a final extention of 10 min. at 72°C) with the attB1 (5'-GGGACAAGTTTGTACAAAAAAGCAGGCT-3') and the attB2 (5'-ggggaccactttgtacaagaaagctgggt-3') were performed in a 50 µl reaction volume. PCR product was gel purified and 50 ηg insert was recombined into 150 ηg pDONR7 vector with the clonase BP enzyme. The vector was transformed into electrocompotent DH5α E.coli cells and plasmids containing the correct insert were isolated and 100 ηg of the pDONR7 with the DMR6 amplicon were used in the LR reaction to recombine the insert in two opposite direction into 150 ηg pHellsgate8 vector. After transformation into E.coli, Spectomycin resistant clones were selected and the isolated plasmids were verified by a Notl digest for the right insert size and by colony PCR with a single internal primer for At5G24530 (DfragmentF: 5'-gagaagtgggatttaaaatagaggaa-3'), if the inserts was inserted twice in opposite direction an amplicon of 1420 bp could be detected. Correct pHellsgate8 plasmids with the double insert in opposite directions were transformed into electrocompotent Agrobacterium strain, C58C1. Plasmids were isolated from the Agrobacterium and retransformed into the *E.coli* to confirm the right size of the plasmid and the insert by Notl digestion. The reconfirmed Agrobacterium strains were used for the floral dip transformation of the Col-0 and L*er eds1-2* plants. The developed seeds were screened for Kanamycin resistance on ½x GM plates, the T₁ seedlings were transferred and the next generation of seeds the T₂ was analysed for DMR6 expression and *H. parasitica* susceptibility.

### Gene expression profiling of the dmr6 mutant

Total RNA was isolated as described above. mRNA was amplified with the MessageAmp aRNA kit (Ambion). CATMA array (Crowe et al., 2003) slides containing approximately 25.000 gene specific tags were hybridized according to standardized conditions described by de Jong et al. (de Jong M., van Breukelen B., Wittink,F.R., Menke,F.L., Weisbeek,P.J., and Van den Ackerveken G. (2006). Membrane-associated transcripts in Arabidopsis; their isolation and characterization by DNA microarray analysis and bioinformatics. Plant J. 46, 708-721). For quantitative PCR, cDNA templates were generated as described previously. Cycle thresholds were determined per transcript in triplicate using the ABI PRISM 7700 sequence detection system (Applied Biosystems, Foster City, CA, USA) using SYBR Green I (Applied Biosystems, Foster City, CA, USA) as reporter dye. Primer sets for the transcripts are *DMR6* (QDMR6F:5'-TGTCATCAACATAGGTGACCAG-3' and QDMR6R: 5'-CGATAGTCACGGATTTTCTGTG-3'), At1g14880 (QAt1g14880F:5'-CTCAAGGAGAATGGTCCACA-3' and QAt1g14880R: 5'-CGACTTGGCCAAATGTGATA-3'), At4g14365 (QAt4g14365F: 5'-TGGTTTTCTGAGGCATGTAAA-3' and QAt4g14365R:5'-AGTGCAGGAACATTGGTTGT-3'), ACD6 (QACD6F:5'-TGGACAGTTCTGGA GCAGAT-3' and QACD6R: 5'-CAACTCCTCCGCTGTGAG-3'), PR-5 (QPR-5F:5'-GGCAAATATCTCCAGTATTCACA-3' and QPR-5R: 5'-GGTAGGGCAAT TGTTCCTTAGA-3'), PR-2 (QPR-2 F:5'-AAGGAGCTTAGCCTCACCAC-3' and QPR-2R: 5'-GAGGGAAGCAAGAATGGAAC -3'), PR-1 (QPR-1F:5'-GAACACGTGCAATGGAGTTT-3'and QPR-1R: 5'-GGTTCCACCATTGTTACACCT-3') and ACT-2 (QACT2 F:5'- AATCACAGCACTTGCACCA-3' and QACT2R: 5'- GAGGGAAGCAAGAATGGAAC-3') generating 100 base pair fragments.

### Results

### Characterization of the gene responsible for pathogen resistance in the dmr6 mutant

Van Damme et al., 2005, supra disclose a dmr6 mutant that is resistant to *H. parasitica.* The level of resistance can be examined by counting the number of sporangiophores per seedling seven day post inoculation with the *H. parasitica* (isolate Waco9 or Cala2, obtainable from Dr. G. Van den Ackerveken, Plant-Microbe Interactions Group, University of Utrecht, Utrecht, NL). The parental line, L*er eds1-2* (Parker et al., 1996, Plant Cell 8:2033-2046), which is highly susceptible, is used as a positive control (and is set at 100%).

The reduction in sporangiophore formation on the infected *dmr6* mutants compared to seedlings of the parental lines is shown in **Fig. 6a****,** wherein the results of the quantification of *Hyaloperonospora parasitica,* Waco9 sporulation (sporangiophores/ seedling) on the downy mildew resistant *dmr6-1* mutant, back-crossed twice to the parental line L*er eds1-2,* and on mutant *dmr6-2* (flag- 445009 T-DNA line) compared to the control lines is shown.

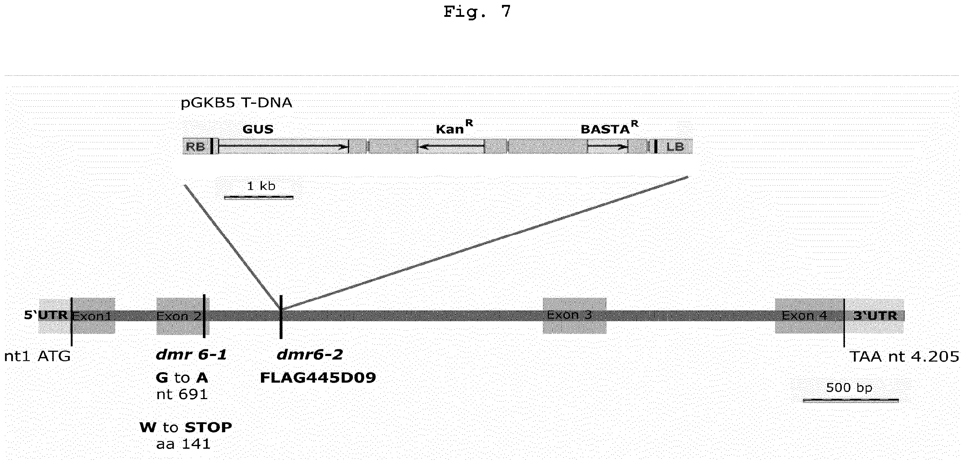

According to the invention, the gene responsible for resistance to *H. parasitica* in the *dmr6* mutants of van Damme et al., 2005, supra, has been cloned by a combination of mapping and sequencing of candidate genes. Previously, the recessive *dmr6* mutation was mapped near the nga139 marker on chromosome 5 to a region encompassing 74 genes. Fine mapping linked the dmr6 locus to a mapping interval containing the BACs T13K7 and K18P6 between the markers At5g24420 and At5g24590 located in the corresponding genes. This allowed the *dmr6* interval to be confined to a region of 18 candidate genes. Comparative sequence analysis of the 18 genes in *dmr6* and the parental line, L*er eds1-2* revealed a point mutation in the second exon of the At5g24530 gene. This single base change of G to A, typical for an EMS mutation, changes a TGG a (trp codon) to a TGA (premature stop codon) at nucleotide position 691 of the coding sequence (**Figure 7**). The early stop codon truncates the predicted oxidoreductase enzyme of 342 aa at position 141 before the conserved catalytic domain suggesting that *dmr6* is a null-allele. The At5g24530 coding sequence (**Figure 2**) is predicted to encode a protein with a mass of 39.4 kDa. No biological role has so far been described for At5g24530.

### At5g24530 is DMR6

A second allele, *dmr6-2,* was identified in a T-DNA insertion line (FLAG_445D09) from the mutant collection from INRA, Versailles. The presence and location of the T-DNA insert in the second intron of At5g24530 (**Figure 7**) was confirmed by PCR and sequence analysis (data not shown). Progeny of the Flag_445D09 line homozygous for the T-DNA insertion was resistant to *H. parasitica* isolate Waco9, whereas the parental line (Ws-4) was susceptible (**Figure 6a**). The At5g24530 transcript could be amplified by RT-PCR using primers in exon 2 and 3 in Ws-4, but not in the homozygous *dmr6-2* line (data not shown), indicating that *dmr6-2* can be considered a second null-allele.

To corroborate the idea that At5g24530 is required for susceptibility to *H. parasitica* the *dmr6-1* mutant was transformed with the cDNA from At5g24530 cloned under control of the 35S promoter. In five independent *dmr6-1* T₂ seedlings the strong overexpression of At5g24530 was confirmed by RT-PCR (data not shown). All T3 lines, homozygous for the transgene, showed restoration of susceptibility to *H. parasitica* isolate Cala2 (**Figure 6b**), confirming that At5g24530 is *DMR6.* The complementation, together with the identification of two independent *dmr6* mutants clearly indicates that a functional *DMR6* gene is required for susceptibility to *H. parasitica.*

### DMR6 is transcriptionally activated during H. parasitica infection

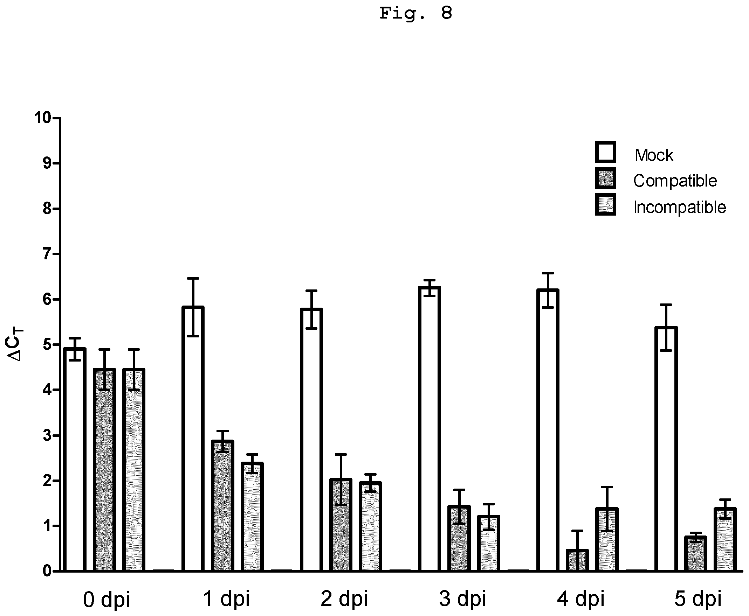

To study the expresssion of *DMR6* during infection with *H. parasitica* relative transcript levels were measured by quantitative PCR at six different time points from 0 days (2 hours) post inoculation to 5 days post inoculation (dpi) (Figure 8). RNA was isolated from ten day old L*er* seedlings that were spray inoculated with water (mock), compatible, or incompatible *H. parasitica* isolate. At 2 hours post inoculation (0 dpi) the levels of DMR6 transcripts were equal in the different treatments. Starting from 1 dpi, the level of *DMR6* transcript was significantly increased in both the compatible and incompatible interaction compared to mock-treated seedlings. The *DMR6* transcript level was slightly but significantly higher at 1 dpi in the incompatible interaction (ΔCT of 3.5, approximately 11 fold induction) than in the compatible (ΔCT of 3.0, approximately 8 fold induction). The expression level increased further in time to reach a stable high level at 4-5 dpi. At these time points the *DMR6* transcript level was higher in the compatible than in the incompatible interaction. The elevated *DMR6* transcript levels during compatible and incompatible *H. parasitica* interactions suggest a role of *DMR6* in plant defence. The defence-associated expression of *DMR6* could be confirmed in our three enhanced-defence mutants, *dmr3, dmr4,* and *dmr5* (Van den Ackerveken et al., unpublished). Furthermore, in silico analysis of *DMR6* levels in the Genevestigator Mutant Surveyor (Zimmermann,P., Hennig,L., and Gruissem,W. (2005). Gene-expression analysis and network discovery using Genevestigator. Trends Plant Sci. 10,407-409) showed that the gene is strongly induced in the pathogen resistant mutants *mpk4* and *cpr5.* In the *cpr5*/*npr1* double mutant the DMR6 transcript level remained high indicating that the induction of *DMR6* expression is mostly *NPR1* independent. Salicylic acid appears to be an important signal in the induction of *DMR6* expression during senescence as *nahG* transgenic plants (expressing the bacterial salicylate hydroxylase gene) showed only low levels of *DMR6* transcript.

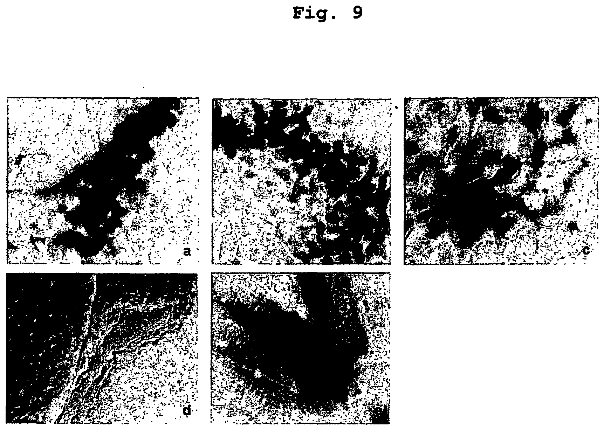

To investigate in more detail how the expression of *DMR6* is activated during biotic and abiotic stress, *DMR6* reporter lines were generated. The localisation of *DMR6* expression was studied in transgenic Col-0 and L*er eds1-2* plants containing the *DMR6* promoter linked to the *uidA* (β-glucuronidase, GUS) reporter gene (pDMR6::GUS). To visualise both *H. parasitica* hyphal growth, by staining with trypan blue, as well as GUS activity, magenta-Xgluc was used as a (β-glucuronidase substrate yielding a magenta precipitate. In uninfected plants no GUS expression could be detected in the different plant organelles; roots, meristem, flower, pollen and seed. The expression of *DMR6* was induced in the compatible interactions, L*er eds1-2* infected with Cala2 (**Figure 9a**), and Col-0 infected with isolate Waco9 (**Figure 9b**). GUS expression was also induced in the incompatible interaction L*er eds1-2* inoculated with isolate Emoy2 (**Figure 9c**). As shown in **figure 9a** and **9b** *DMR6* expression was confined to the cells in which *H. parasitica* had formed haustoria. Plant cells containing the most recently formed haustoria did not show detectable levels of GUS activity (**Figure 9a****,** indicated by asterisk). During the incompatible interaction (**Figure 9c**) activity of the *DMR6* promoter could only be detected in the cells that were in contact with the initial invading hyphae. In death cells, resulting from the hypersensitive response (HR, visualized by trypan blue staining indicated in **Figure 9c** by asterisk) no GUS activity could be detected, possibly due to protein degradation in these cells. To test if the *DMR6* expression in haustoria-containing cells is caused by a wound-like response, seedlings were wound by incision with scissors and stained for GUS activity 3 days later. No detectable promoter *DMR6* GUS expression was seen, indicating that the expression of *DMR6* is not induced by wounding (**Figure 9d**). Furthermore the local induction of *DMR6* expression was tested in response to treatment with benzothiadiazole (BTH), a functional analogue of salicylic acid (SA). At 3 days post BTH treatment GUS activity was mainly localized in the newly formed, but not in the mature leaves (**Figure 9e**)**.** Analysis of pDMR6::GUS lines confirm the expression data described above and highlights the strictly localized induction of *DMR6* in response to *H. parasitica* infection.

### The dmr6-1 mutant constitutively expresses defence associated transcripts

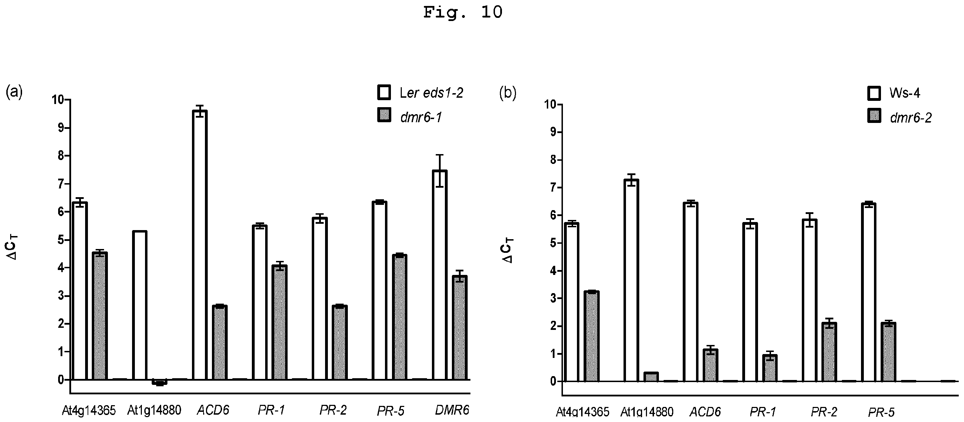

To elucidate how the lack of *DMR6* results in *H. parasitica* resistance, the transcriptome of the *dmr6-1* mutant compared to the L*er eds1-2* parental line was analysed. Probes derived from mRNA of the above-ground parts of 14 day old *dmr6-1* and L*er eds1-2* seedlings were hybridised on whole genome CATMA micro arrays. A total of 58 genes were found to be significantly differentially expressed in *dmr6-1,* of which 51 genes had elevated and 7 genes had reduced transcript levels. A pronounced set of the 51 induced transcripts have been identified as genes associated with activated plant defence responses, e.g., ACD6, PR-5, PR-4/HEL and PAD4. These data indicate that the loss of *DMR6* results in the activation of a specific set of defence-associated transcripts. The finding that *DMR6* is among the dmr6-1-induced genes corroborates the idea that *DMR6* is defence-associated. To test if the induced expression of the defence-associated genes was due to the loss of *DMR6* and not due to additional ethane methyl sulfonate (EMS) mutations remaining in the backcrossed *dmr6-1* mutant the transcript level of a selection of genes (At4g14365, At1g14880, *ACD6, PR-1, PR-2* and *PR-5*) was verified by quantitative PCR in both the *dmr6-1* and *dmr6-2* mutant (**Figure 10**). We could only test *DMR6* transcript levels in the *dmr6-1* mutant (**Figure 10a**) as the *dmr6-2* mutant (**Figure 10b**) has a T_DNA insertion disrupting the *DMR6* transcript. The induction of *DMR6* as observed in the micro array analysis was confirmed by Q-PCR in dmr6-1 compared to L*er eds1-2* (**Figure 10a**). **Figure 10a** and **b** show that all six selected genes were elevated in both *dmr6* mutants compared to the parental lines. The observed elevated expression of the selected defence-associated genes in the *dmr6* mutants indicates that lack of *DMR6* activates a plant defence response. The activation of this set of defence-associated transcripts could be the primary cause of resistance to *H. parasitica* in the *dmr6* mutants.

### EXAMPLE 2

### Identification of DMR6 orthologs in crops

### 1. Screening of libraries on the basis of sequence homology

The nucleotide and amino acid sequences of the DMR6 coding sequence and protein of *Arabidopsis thaliana* are shown in **Fig. 2****.** Public libraries of nucleotide and amino acid sequences were compared with the sequences of **Fig. 2****.** This comparison resulted in identification of the complete DMR6 coding sequences and predicted amino acid sequences in *Aquilegia species, Citrus sinensis, Coffea canephora, Cucumis sativus, Gossypium hirsitum, Lactuca sativa, Medicago truncatula, Oryza sativa (3), Populus trichocarpa (2), Solanum lycopersicum (2), Sorghum bicolor, Spinacia oleracea, Vitis vinifera, Zea mays, and Zingiber officinale.* The sequence information of the orthologous proteins thus identified is given in **Table 1** and visualized in an multiple alignment in **Fig. 1****.** For many other plant species orthologous DNA fragments could be identified by BlastX as reciprocal best hits to the *Arabidopsis* or other plant DMR6 protein sequences.

### 2. Identification of orthologs by means of heterologous hybridisation

The DMR6 DNA sequence of *Arabidopsis thaliana* as shown in **Fig. 2** is used as a probe to search for homologous sequences by hybridization to DNA of any plant species using standard molecular biological methods. Using this method orthologous genes are detected by southern hybridization on restriction enzyme-digested DNA or by hybridization to genomic or cDNA libraries. These techniques are well known to the person skilled in the art. As an alternative probe the DMR6 DNA sequence of any other more closely related plant species can be used as a probe.

### 3. Identification of orthologs by means of PCR

For many crop species, partial DMR6 mRNA or gene sequences are available that are used to design primers to subsequently PCR amplify the complete cDNA or genomic sequence. When 5' and 3' sequences are available the missing internal sequence is PCR amplified by a DMR6 specific 5' forward primer and 3' reverse primer. In cases where only 5', internal or 3' sequences are available, both forward and reverse primers are designed. In combination with available plasmid polylinker primers, inserts are amplified from genomic and cDNA libraries of the plant species of interest. In a similar way, missing 5' or 3' sequences are amplified by advanced PCR techniques; 5'RACE, 3' RACE, TAIL-PCR, RLM-RACE or vectorette PCR.

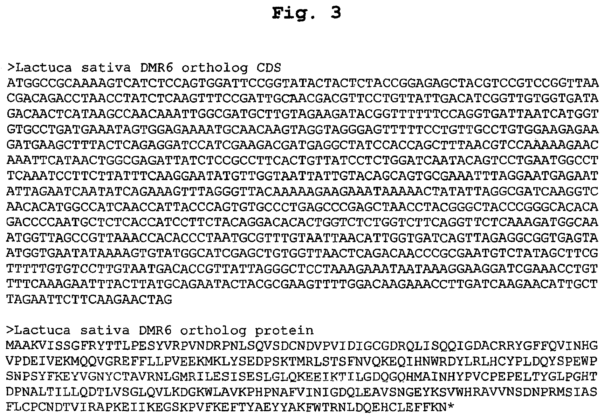

As an example the sequencing of the *Lactuca sativa* (lettuce) DMR6 cDNA is provided. From the Genbank EST database at NCBI several *Lactuca* DMR6 ESTs were identified using the tblastn tool starting with the *Arabidopsis* DMR6 amino acid sequence. Clustering and alignment of the ESTs resulted in a consensus sequence for a 5' DMR6 fragment. To obtain the complete lettuce DMR6 cDNA the RLM-RACE kit (Ambion) was used on mRNA from lettuce seedlings. The 3' mRNA sequence was obtained by using two primers that were designed in the 5' DMR6 consensus sequence derived from ESTs (Lsat_dmr6_fw1: CGATCAAGGTCAACACATGG, and Lsat_dmr6_fw2: TCAACCATTACCCAGTGTGC) and the 3'RACE primers from the kit. Based on the assembled sequence new primers were designed to amplify the complete DMR6 coding sequence from cDNA to provide the nucleotide sequence and derived protein sequence as presented in **Figure 3****.**

The complete DMR6 coding sequences from more than 10 different plants species have been identified from genomic and EST databases. From the alignment of the DNA sequences, conserved regions in the coding sequence were selected for the design of degenerate oligonucleotide primers (for the degenerate nucleotides the abbreviations are according to the IUB nucleotide symbols that are standard codes used by all companies synthesizing oligonucleotides; G = Guanine, A = Adenine, T = Thymine, C = Cytosine, R = A or G, Y = C or T, M = A or C, K = G or T, S = C or G, W = A or T, B = C or G or T, D = G or A or T, H = A or C or T, V = A or C or G, N = A or C or G or T).

The procedure for obtaining internal DMR6 cDNA sequences of a given plant species is as follows:
1. mRNA is isolated using standard methods,
2. cDNA is synthesized using an oligo dT primer and standard methods,
3. using degenerate forward and reverse oligonucleotides a PCR reaction is carried out,
4. PCR fragments are separated by standard agarose gel electrophoresis and fragments of the expected size are isolated from the gel,
5. isolated PCR fragments are cloned in a plasmid vector using standard methods,
6. plasmids with correct insert sizes, as determined by PCR, are analyzed by DNA sequencing,
7. Sequence analysis using blastX reveals which fragments contain the correct internal DMR6 sequences,
8. The internal DNA sequence can then be used to design gene- and species- specific primers for 5' and 3' RACE to obtain the complete DMR6 coding sequence by RLM-RACE (as described above).

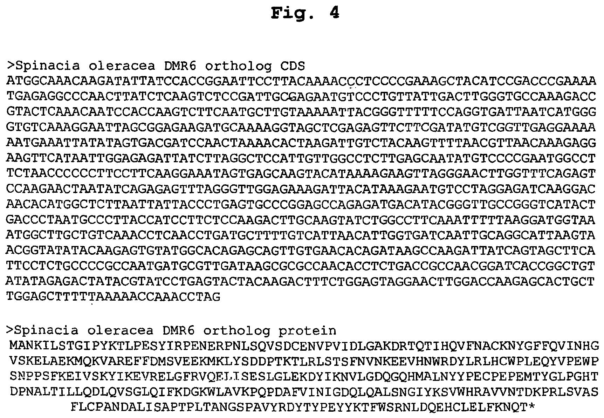

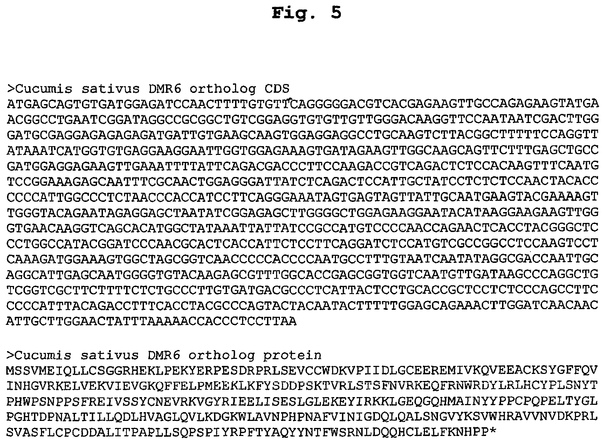

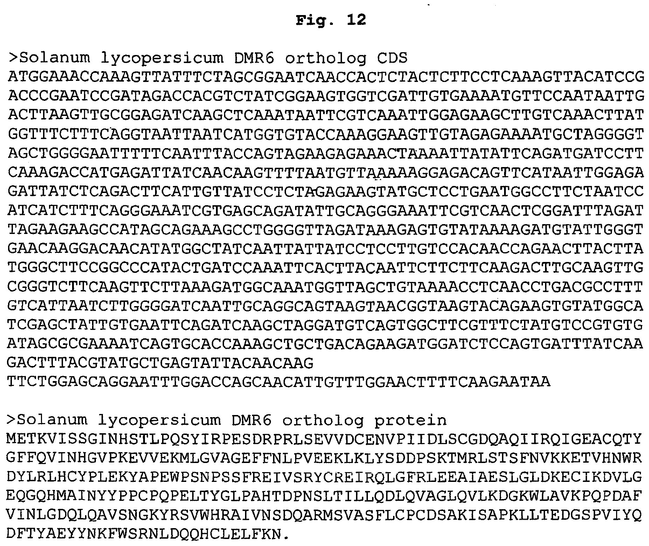

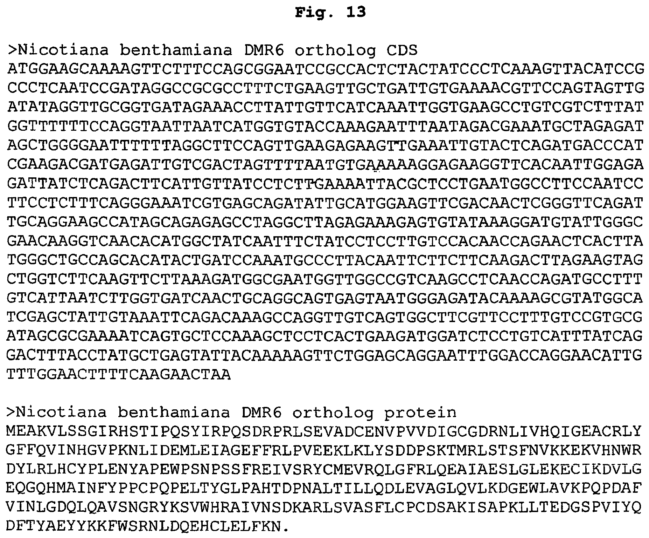

As an example the sequencing of the *Cucumis sativus* (cucumber) DMR6 cDNA is provided. For cucumber several primer combinations between the following primers were successful in amplifying a stretch of internal coding sequence from cDNA; forward primers dmr6_deg_fw1B (TTCCAGGTDATTAAYCAYGG), dmr6_deg_fw2B CATAAYTGGAGRGAYTAYCT), dmr6_deg_fw3B (GARCAAGGRCARCAYATGGC) and dmr6_deg_fw4 (AATCCTCCTTCHTTCAAGGA) and reverse primers dmr6_deg_rv3B (AGTGCATTKGGGTCHGTRTG), dmr6_deg_rv4 (AATGTTRATGACAAARGCAT) and dmr6_deg_rv5 (GCCATRTGYTGYCCTTGYTC). After cloning and sequencing of the amplified fragments cucumber DMR6-specific primers were designed for 5' RACE (Cuc_dmr6_rv1: TCCGGACATTGAAACTTGTG and Cuc_dmr6_rv2: TCAAAGAACTGCTTGCCAAC) and 3' RACE (Cuc_dmr6_fw1: CGCACTCACCATTCTCCTTC and Cuc_dmr6_fw2: GGCCTCCAAGTCCTCAAAG). Finally the complete cucumber DMR6 cDNA sequence was amplified and sequenced (**Figure 5**). A similar approach was a used for spinach, *Spinacia oleracea* (**Figure 4**), *Solanum lycopersicum* (**Figure 12**) and *Nicotiana benthamiana* (**Figure 13**).

Orthologs identified as described in this example can be modified using well-known techniques to induce mutations that reduce the DMR6 expression or activity, to obtain non-genetically modified plants resistant to Fungi or Oomycota. Alternatively, the genetic information of the orthologs can be used to design vehicles for gene silencing, and to transform the corresponding crop plants to obtain plants that are resistant to *Oomycota.*

### EXAMPLE 3

### Mutation of seeds

Seeds of the plant species of interest are treated
with a mutagen in order to introduce random point mutations
in the genome. Mutated plants are grown to produce seeds and
the next generation is screened for the absence of reduction of DMR6 transcript levels or activity. This is achieved by monitoring the level of DMR6 gene expression, or by searching for nucleotide changes (mutations) by the TILLING method, by DNA sequencing, or by any other method to identify nucleotide changes. The selected plants are homozygous or are made homozygous by selfing or inter-crossing. The selected homozygous plants with absent or reduced DMR6 transcript activity are tested for increased resistance to the pathogen of interest to confirm the increased disease resistance.

### EXAMPLE 4

### Transfer of a mutated allele into the background of a desired crop

Introgression of the desired mutant allele into a crop is achieved by crossing and genotypic screening of the mutant allele. This is a standard procedure in current-day marker assistant breeding of crops.

### EXAMPLE 5

### Use of the DMR6 promotor for pathogen-induced gene expression and the generation of disease resistant plants

Precise control of transgene expression is pivotal to the engineering of plants with increased disease resistance. In the past, constitutive overexpression of transgenes frequently has resulted in poor quality plants. It has therefor been suggested to use pathogen-inducible promotors, by which the transgenes are expressed only when and where they are needed - at infection sites.

Local and inducible expression of engineered genes, e.g. master switch genes, elicitor or Avr genes, anti-microbial genes, or toxic genes, results in the activation of defence or cell death that will lead to pathogen resistance, such as described by Gurr and Rushton (Trends in Biotechnology 23: 275-282, 2005). A good example is provided by De wit (Annu. Rev. Phytopathol. 30: 391-418, 1992) who proposes the use of the Avr9-Cf9 combination to achieve induced cell death leading to disease resistance. The tissue-specificity and inducibility of expression is of prime importance for such approaches, as described by Gurr and Rushton (Trends in Biotechnology 23: 283-290, 2005).

According to the present invention, the DMR6 promoter has been demonstrated to show a strong, inducible, localized expression based on promoter-GUS analysis. Thus, the DMR6 promotor is very suitable for engineering disease resistance in transgenic plants. The DMR6 promoter consists of a region of 2.5 kb that is upstream of the Arabidopsis DMR6 coding sequence (ATG start codon) and includes the 5'UTR (as depicted in **Figure 11**). This pathogen-inducible promotor is then used to engineer suitable transgene constructs, using standard techniqus known the person skilled in the art.

Using orthologous DNA sequences from a given plant species primers are designed for PCR. These are then used to screen genomic libraries of the plant species of interest to identify the genomic clones that contain the DMR6 ortholog with its promoter and regulatory sequences. Alternatively, the genomic clones are isolated by screening a library with a labelled PCR fragment corresponding to the DMR6 orthologous gene. Sequencing reveals the nucleotide sequence of the promoter. The region of 2-5 kb upstream the DMR6 orthologous coding sequence (ATG start codon), so including the 5'UTR, is then amplified by PCR to engineer transgene constructs for plant transformation.

### EXAMPLE 6

This example demonstrates the complementation of mutant *dmr6-1* in *Arabidopsis thaliana* by *DMR6* orthologs from 4 different crop species. For this, *DMR6* orthologs of *Cucumis sativa* (Cs), *Spinacia oleracea* (So), *Lactuca sativa* (Ls) and *Solanum lycopersicum* (Sl) were cloned into a plant expression vector under the control of the 35S promoter and, subsequently, this vector was transformed into a *Arabidopsis thaliana* mutant *dmr6-1.*

Briefly, mRNA was isolated using standard methods and cDNA was synthesized using an oligo dT primer and standard methods. Subsequently, PCR fragments were generated using primer pairs for each crop as depicted in **table 3** below. The generated PCR products were cloned into a pENTR/D-TOPO vector using the pENTR/D-TOPO cloning kit from Invitrogen and resulting plasmids with correct insert sizes, as determined by PCR, were analyzed by DNA sequencing. Recombination to the pB7WG2,0 vector was done using LR clonase II from Invitrogen and the resulting plasmids were analyzed by PCR and digestion with restriction enzymes. Suitable plasmids were transformed into *Agrobacterium tumefaciens* C58C1 PGV2260 and plasmids from Agrobacterium were analyzed by PCR and digestion with restriction enzymes.

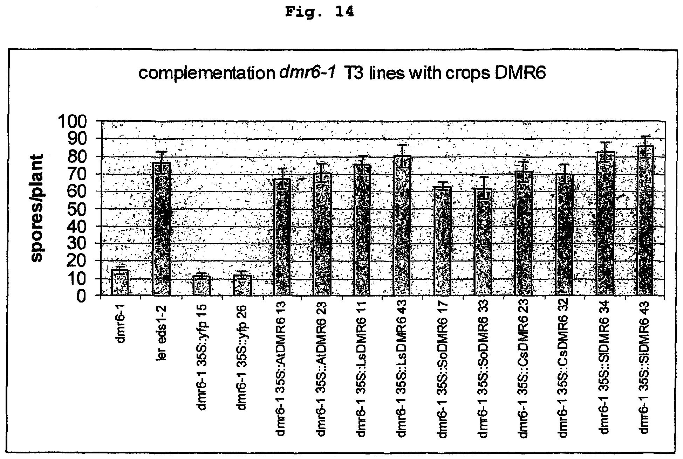

*Arabidopsis thaliana* dmr6-1 plants were transformed with the above constructs by dipping into *Agrobacterium* solution and overexpression of crops DMR6 in Arabidopsis T1 plants is verified by RT-PCR using the crops DMR6 cloning primers (**table 3**). Finally, Arabidopsis T2 and T3 plants were infected with *Hyaloperonospora parasitica* Cala2 to confirm complementation. The results are shown in **figure 14****.**

As shown in **figure 14****,** all *DMR6* orthologs tested were capable of complementing *Arabidopsis thaliana* mutant *dmr6-1* indicating that the *DMR6* orthologs identified encode DMR6 proteins with a similar functionality as *Arabidopsis thaliana* DMR6.

### TABLES

Table 1 lists the GI numbers (Genlnfo identifier) and Genbank accession number for Expressed Sequence Tags (ESTs) and mRNA or protein sequences of the Arabidopsis DMR6 mRNA and orthologous sequences from other plant species. A GI number (genlnfo identifier, sometimes written in lower case, "gi") is a unique integer which identifies a particular sequence. The GI number is a series of digits that are assigned consecutively to each sequence record processed by NCBI. The GI number will thus change every time the sequence changes. The NCBI assigns GI numbers to all sequences processed into Entrez, including nucleotide sequences from DDBJ/EMBL/GenBank, protein sequences from SWISS-PROT, PIR and many others. The GI number thus provides a unique sequence identifier which is independent of the database source that specifies an exact sequence. If a sequence in GenBank is modified, even by a single base pair, a new GI number is assigned to the updated sequence. The accession number stays the same. The GI number is always stable and retrievable. Thus, the reference to GI numbers in the table provides a clear and unambiguous identification of the corresponding sequence.

**Table 1**

| **Species** | **Common name** | **Detail** | **GI number** | **Genbank** |
|---|---|---|---|---|
| *Arabidopsis thaliana* | Thale cress | mRNA | 42568064 | NM_122361 |
| *Aquilegia-sp* | Aquilegia | ESTs | 75461114 | DT768847.1 |
| | | | 74538666 | DT745001.1 |
| | | | 74562677 | DT760187.1 |
| | | | 75461112 | DT768846.1 |
| | | | 74562675 | DT760186.1 |
| *Citrus sinensis* | Sweet Orange | ESTs | 5793134 | CX672037.1 |
| | | | 57933368 | CX673829.1 |
| | | | 63078039 | CX309185.1 |
| *Coffea canephora* | Coffea | ESTs | 82485203 | DV705375.1 |
| | | | 82458236 | DV684837.1 |
| | | | 82461999 | DV688600.1 |
| | | | 82487627 | DV707799.1 |
| *Gossypium hirsutum* | Cotton | ESTs | 109842586 | DW241146.1 |
| | | | 48751103 | CO081622.1 |
| *Sorghum bicolor* | Sorghum | ESTs | 45992638 | CN150358.1 |
| | | | 57813436 | CX614669.1 |
| | | | 45985339 | CN145819.1 |
| | | | 57821006 | CX622219.1 |
| | | | 45989371 | CN148311.1 |
| | | | 57821495 | CX622708.1 |
| | | | 45959033 | CN130459.1 |
| | | | 45985193 | CN145752.1 |
| | | | 18058986 | BM322209.1 |
| | | | 45958822 | CN130381.1 |
| | | | 30164583 | CB928312.1 |
| *Medicago truncatula* | Barrel medic | Genome draft | | MtrDRAFT_AC119415g1v1 |
| | | protein | 92878635 | ABE85154 |
| *Oryza sativa 1* | Rice | Genome | | OSJNBb0060l05.4 |
| | | protein | 18057095 | AAL58118.1 |
| *Oryza sativa 2* | | mRNA | 115450396 | NM_001055334 |
| | | protein | 115450397 | NP_001048799 |
| *Oryzasativa 3* | | mRNA | 115460101 | NM_001060186 |
| | | protein | 115460102 | NP_001053651 |
| *Populus trichocarpa 1* | Poplar | Genome: LG-XII:3095392-3103694 | | |
| | | protein: Poptr1_1:569679, eugene3.00120332 | | |
| *Populus trichocarpa 2* | Poplar | Genome: LG_XV:201426-209590 | | |
| | | protein: Poptr1_1:732726, estExt_Genewise1_v1.C_LG_XV0083 | | |
| *Solanum lycopersicum 1* | Tomato | ESTs | 62932307 | BW689896.1 |
| | | | 58229384 | BP885913.1 |
| | | | 117682646 | DB678879.1 |
| | | | 5894550 | AW035794.1 |
| | | | 117708809 | DB703617.1 |
| | | | 62934028 | BW691617.1 |
| | | | 15197716 | BI422913.1 |
| | | | 4381742 | AI486371.1 |
| | | | 5601946 | AI896044.1 |
| | | | 4387964 | AI484040.1 |
| | | | 4383017 | AI487646. |
| | | | 5278230 | AI780189.1 |
| | | | 12633558 | BG133370.1 |
| | | | 76572794 | DV105461.1 |
| | | | 117692514 | DB718569.1 |
| | | | 4385331 | AI489960.1 |
| | | | 4383253 | AI487882.1 |
| | | | 4384827 | AI489456.1 |
| *Solanum lycopersicum 2* | Tomato | ESTs | 47104686 | BT013271.1 |
| | | | 14685038 | BI207314.1 |
| | | | 14684816 | BI207092.1 |
| *Zea mays* | Maize | ESTs | 110215403 | EC897301.1 |
| | | | 76291496 | DV031064.1 |
| | | | 91050479 | EB160897.1 |
| | | | 91874282 | EB404239.1 |
| | | | 110540753 | EE044673.1 |
| | | | 78111856 | DV530253.1 |
| | | | 94477588 | EB706546.1 |
| | | | 71441483 | DR822533.1 |
| | | | 78111699 | DV530096.1 |
| | | | 78107139 | DV525557.1 |
| | | | 76017449 | DT944619.1 |
| | | | 91048249 | EB158667.1 |
| | | | 78104908 | DV523326.1 |
| | | | 78088214 | DV516607.1 |
| | | | 76291495 | DV031063.1 |
| | | | 71441482 | DR822532.1 |
| | | | 78088213 | DV516606.1 |
| *Vitis vinifera* | Grape | ESTs | 33396402 | CF202029.1 |
| | | | 33399765 | CF205392.1 |
| | | | 45770972 | CN006824.1 |
| | | | 45770784 | CN006636.1 |
| | | | 45770528 | CN006380.1 |
| | | | 45770631 | CN006483.1 |
| | | | 33400623 | CF206250.1 |
| | | | 33396335 | CF201962.1 |
| | | | 30134763 | CB920101.1 |
| | | | 30305300 | CB982094.1 |
| | | | 71857419 | DT006474.1 |
| | | | 30305235 | CB982029.1 |
| *Zingiber officinale* | Ginger | ESTs | 87108948 | DY375732.1 |
| | | | 87095447 | DY362231.1 |
| | | | 87095448 | DY362232.1 |
| | | | 87094804 | DY361588.1 |
| | | | 87095449 | DY362233.1 |
| | | | 87094803 | DY361587.1 |
| *Lactuca sativa* | Lettuce | Sequence described in this patent application | | |
| *Spinacia oleracea* | Spinach | Sequence described in this patent application | | |
| *Cucumis sativus* | Cucumber | Sequence described in this patent application | | |
| Nicotiana benthamiana | Tabac | Sequence described in this patent application | | |

**Table 2**

| Primer sequences of insertion/deletion markers (size difference in brackets) used in the mapping and cloning of the DMR6 gene. | | | | |
|---|---|---|---|---|
| Name primer | Gene | INDEL/ enzyme | Forward primer | Reverse primer |
| IND_MOP9 | At5G24210 | | | |
| IND_K16H17 | At5g24420 | | | |
| IND_T4C12 | At5g24820 | | | |
| IND_T11H3 | At5g24950-60 | | | cggcttttaacaacatattttcca |
| IND_F21J6 | At5g25270 | | | |
| M450 | At5G24450 | 18 | | |
| M490 | At5g24490 | Taql | | |
| M525 | At5g24520-30 | Taql | | tcaagatcttcatattctcattcca |
| M545 | At5G24540/50 | 41 | | |
| M555 | At5G24550/60 | 14 | | |
| M470 | At5g24470 | Hphl | | |
| M590 | At5g24590 | Pdml | | |

**Table 3**

| Primer pairs for cloning dmr6 orthologs in a suitable plant expression vector | | |
|---|---|---|
| *Arabidopsis thaliana* | AtDMR6_fw | CACCATGGCGGCAAAGCTGATA |
| | AtDMR6UTR_rv | GACAAACACAAAGGCCAAAGA |
| *Cucumis sativa* | cuc_fw | CACCATGAGCAGTGTGATGGAGAT |
| | cucUTR_rv | TGGGCCAAAAAGTTTATCCA |
| *Spinacia oleracea* | spi_fw | |
| | spiUTR_rv | TTGCTGCCTACAAAAGTACAAA |
| *Lactuca sativa* | Leat_fw | CACCATGGCCGCAAAAGTCATCTC |
| | LsatUTR_rv | CATGGAAACACATATTCCTTCA |
| *Solanum lycopersicum* | Slyc1dmr6_fw | |
| | Slyc1dmr6UTR_rv | GGGACATCCCTATGAACCAA |

## Claims

1. Melon plant which is resistant to *Pseudoperonospora cubensis,* **characterized in that** the plant has a reduced level or complete absence of DMR6 protein as compared to the plant that is not resistant to the pathogen wherein said plant has a mutation in its *DMR6* gene resulting in a reduced DMR6 expression as compared to the wild-type DMR6 gene wherein no such mutation is present.

2. Method for obtaining a melon plant which is resistant to *Pseudoperonospora cubensis* comprising reducing the endogenous level of DMR6 protein in the plant by mutation of the DMR6 gene of the plant.

3. Method according to claim 2, wherein the mutation is effected by mutagenic treatment of the plant, in particular with mutagens or radiation.

4. Method according to claim 2, wherein reducing the endogenous level in the plant is achieved by reducing the expression of the *DMR6* gene of the plant by gene silencing or RNAi.

## Patentansprüche

1. Melonenpflanze, die gegenüber *Pseudoperonospora cubensis* resistent ist,
**dadurch gekennzeichnet,**
**dass** die Pflanze ein reduziertes Niveau oder vollständiges Fehlen des DMR6-Proteins im Vergleich zu der Pflanze aufweist, die gegenüber dem Pathogen nicht resistent ist,
wobei die Pflanze in ihrem DMR6-Gen eine Mutation aufweist, die im Vergleich zu dem Wildtyp-DMR6-Gen, in dem keine derartige Mutation vorhanden ist, in einer reduzierten DMR6-Expression resultiert.

2. Verfahren zur Erzielung einer Melonenpflanze, die gegenüber *Pseudoperonospora cubensis* resistent ist, umfassend:
Reduzieren des endogenen Niveaus des DMR6-Proteins in der Pflanze durch Mutation des DMR6-Gens der Pflanze.

3. Verfahren nach Anspruch 3, wobei die Mutation durch mutagene Behandlung der Pflanze, insbesondere mit Mutagenen oder durch Bestrahlung bewirkt wird.

4. Verfahren nach Anspruch 3, wobei die Reduzierung des endogenen Niveaus in der Pflanze durch Reduzieren der Expression des DMR6-Gens der Pflanze mittels Gen-Stilllegung oder RNAi erreicht wird.

## Revendications

1. Plant de melon qui est résistant à *Pseudoperonospora cubensis,* **caractérisé en ce que**
le plant présente un taux réduit ou une absence complète de la protéine DMR6 comparativement au plant qui n'est pas résistant audit agent pathogène, dans lequel
ledit plant comporte une mutation dans son gène *DMR6* entraînant une expression réduite de la DMR6 comparativement au gène de la DMR6 de type sauvage dans lequel aucune mutation de ce type n'est présente.

2. Procédé d'obtention d'un plant de melon qui est résistant à *Pseudoperonospora cubensis* comprenant la réduction du taux endogène de la protéine DMR6 dans le plant par mutation du gène de la DMR6 du plant.

3. Procédé selon la revendication 3, dans lequel la mutation est effectuée par traitement mutagène du plant, en particulier avec des mutagènes ou des radiations.

4. Procédé selon la revendication 3, dans lequel la réduction du taux endogène dans le plant est obtenue par réduction de l'expression du gène *DMR6* du plant par silençage de gène ou ARNi.
